# EUROPEAN PATENT SPECIFICATION

(11) **EP 1 973 565 B1**
(45) Date of publication and mention of the grant of the patent: **15.05.2013**
(21) Application number: 06821006.1
(22) Date of filing: 24.11.2006
(51) Int. Cl.: A61K 39/12

(54) **MARKED BOVINE VIRAL DIARRHEA VIRUS**
MARKIERTES RINDER-VIRUS-DIARRHÖE-VIRUS
VIRUS DE LA DIARRHÉE VIRALE BOVINE MARQUÉ

(30) Priority: 07.12.2005 US 748312 P
(43) Date of publication of application: 01.10.2008
(73) Proprietor: PAH USA 15 LLC, New York, NY 10017-5755 (US)
(72) Inventor: HUANG, Chichi, Berwyn, PA 19312 (US); SHEPPARD, Michael G., Eltham, VIC 3095 (AU); CAO, Xuemei, Scituate, MA 02066 (US); ZYBARTH, Gabriele, Little Compton, Rhode Island 02837 (US)
(74) Representative: Mannion, Sally Kim
(86) International application number: PCT/IB2006/003412
(87) International publication number: WO 2007/066188

(56) References cited:
- US-A- 6 001 613
- US-A1- 2003 165 520
- GRASSMANN C W ET AL: "Assignment of the multifunctional NS3 protein of bovine viral diarrhea virus during RNA replication: an in vivo and in vitro study." JOURNAL OF VIROLOGY NOV 1999, vol. 73, no. 11, November 1999 (1999-11), pages 9196-9205, XP002474037 ISSN: 0022-538X
- DEREGT D ET AL: "Mapping of two antigenic domains on the NS3 protein of the pestivirus bovine viral diarrhea virus" VETERINARY MICROBIOLOGY, AMSTERDAM, NL, vol. 108, no. 1-2, 15 June 2005 (2005-06-15), pages 13-22, XP004905178 ISSN: 0378-1135
- GU B ET AL: "The RNA helicase and nucleotide triphosphatase activities of the bovine viral diarrhea virus NS3 protein are essential for viral replication." JOURNAL OF VIROLOGY FEB 2000, vol. 74, no. 4, February 2000 (2000-02), pages 1794-1800, XP002474038 ISSN: 0022-538X
- LACKNER T ET AL: "Temporal modulation of an autoprotease is crucial for replication and pathogenicity of an RNA virus." JOURNAL OF VIROLOGY OCT 2004, vol. 78, no. 19, October 2004 (2004-10), pages 10765-10775, XP002474039 ISSN: 0022-538X
- PASICK JOHN: "APPLICATION OF DIVA VACCINES AND THEIR COMPANION DIAGNOSTIC TESTS TO FOREIGN ANIMAL DISEASE ERADICATION" ANIMAL HEALTH RESEARCH REVIEWS, CABI PUBLISHING, GB, vol. 5, no. 2, December 2004 (2004-12), pages 257-262, XP009084982 ISSN: 1466-2523
- GRAHAM D A ET AL: "Antibody responses of naive cattle to two inactivated bovine viral diarrhoea virus vaccines, measured by indirect and blocking ELISAS and virus neutralisation." THE VETERINARY RECORD 28 JUN 2003, vol. 152, no. 26, 28 June 2003 (2003-06-28), pages 795-800, XP009097788 ISSN: 0042-4900
- MAKOSCHEY ET AL: "Evaluation of the induction of NS3 specific BVDV antibodies using a commercial inactivated BVDV vaccine in immunization and challenge trials" VACCINE, BUTTERWORTH SCIENTIFIC. GUILDFORD, GB, vol. 25, no. 32, 20 July 2007 (2007-07-20), pages 6140-6145, XP022162350 ISSN: 0264-410X
- REIMANN I ET AL: "An avirulent chimeric Pestivirus with altered cell tropism protects pigs against lethal infection with classical swine fever virus", VIROLOGY, ACADEMIC PRESS,ORLANDO, US, vol. 322, no. 1, 25 April 2004 (2004-04-25), pages 143-157, XP004500339, ISSN: 0042-6822, DOI: 10.1016/J.VIROL.2004.01.028

### SEQUENCE LISTING

<110> Pfizer Inc.
   Huang, Chichi
   Sheppard, Michael G.
   Cao, Xuemei
   Zybarth, Gabriele
<120> Marked BVDV vaccine
<130> PC33031
<160> 21
<170> PatentIn version 3.2
<210> 1
   <211> 3906
   <212> PRT
   <213> Artificial Bovine Viral Diarrhea virus polyprotein
<220>
   <223> Unprocessed polypeptide from RNA template for BVD virus, NADL isolate
<400> 1
   Leu Lys Pro Gly Pro Leu Phe Tyr Gln Asp Tyr Lys Gly Pro Val Tyr
   1               5                   10                  15
   His Arg Ala Pro Leu Glu Leu Phe Glu Glu Gly Ser Met Cys Glu Thr
               20                  25                  30
   Thr Lys Arg Ile Gly Arg Val Thr Gly Ser Asp Gly Lys Leu Tyr His
           35                  40                  45
   Ile Tyr Val Cys Ile Asp Gly Cys Ile Ile Ile Lys Ser Ala Thr Arg
       50                  55                  60
   Ser Tyr Gln Arg Val Phe Arg Trp Val His Asn Arg Leu Asp Cys Pro
   65                  70                  75                  80
   Leu Trp Val Thr Thr Cys Ser Asp Thr Lys Glu Glu Gly Ala Thr Lys
                   85                  90                  95
   Lys Lys Thr Gln Lys Pro Asp Arg Leu Glu Arg Gly Lys Met Lys Ile
               100                 105                 110
   Val Pro Lys Glu Ser Glu Lys Asp Ser Lys Thr Lys Pro Pro Asp Ala
           115                 120                 125
   Thr Ile Val Val Glu Gly Val Lys Tyr Gln Val Arg Lys Lys Gly Lys
       130                 135                 140
   Thr Lys Ser Lys Asn Thr Gln Asp Gly Leu Tyr His Asn Lys Asn Lys
   145                 150                 155                 160
   Pro Gln Glu Ser Arg Lys Lys Leu Glu Lys Ala Leu Leu Ala Trp Ala
                   165                 170                 175
   Ile Ile Ala Ile Val Leu Phe Gln Val Thr Met Gly Glu Asn Ile Thr
               180                 185                 190
   Gln Trp Asn Leu Gln Asp Asn Gly Thr Glu Gly Ile Gln Arg Ala Met
           195                 200                 205
   Phe Gln Arg Gly Val Asn Arg Ser Leu His Gly Ile Trp Pro Glu Lys
       210                 215                 220
   Ile Cys Thr Gly Val Pro Ser His Leu Ala Thr Asp Ile Glu Leu Lys
   225                 230                 235                 240
   Thr Ile His Gly Met Met Asp Ala Ser Glu Lys Thr Asn Tyr Thr Cys
                   245                 250                 255
   Cys Arg Leu Gln Arg His Glu Trp Asn Lys His Gly Trp Cys Asn Trp
               260                 265                 270
   Tyr Asn Ile Glu Pro Trp Ile Leu Val Met Asn Arg Thr Gln Ala Asn
           275                 280                 285
   Leu Thr Glu Gly Gln Pro Pro Arg Glu Cys Ala Val Thr Cys Arg Tyr
       290                 295                 300
   Asp Arg Ala Ser Asp Leu Asn Val Val Thr Gln Ala Arg Asp Ser Pro
   305                 310                 315                 320
   Thr Pro Leu Thr Gly Cys Lys Lys Gly Lys Asn Phe Ser Phe Ala Gly
                   325                 330                 335
   Ile Leu Met Arg Gly Pro Cys Asn Phe Glu Ile Ala Ala Ser Asp Val
               340                 345                 350
   Leu Phe Lys Glu His Glu Arg Ile Ser Met Phe Gln Asp Thr Thr Leu
           355                 360                 365
   Tyr Leu Val Asp Gly Leu Thr Asn Ser Leu Glu Gly Ala Arg Gln Gly
       370                 375                 380
   Thr Ala Lys Leu Thr Thr Trp Leu Gly Lys Gln Leu Gly Ile Leu Gly
   385                 390                 395                 400
   Lys Lys Leu Glu Asn Lys Ser Lys Thr Trp Phe Gly Ala Tyr Ala Ala
                   405                 410                 415
   Ser Pro Tyr Cys Asp Val Asp Arg Lys Ile Gly Tyr Ile Trp Tyr Thr
               420                 425                 430
   Lys Asn Cys Thr Pro Ala Cys Leu Pro Lys Asn Thr Lys Ile Val Gly
           435                 440                 445
   Pro Gly Lys Phe Gly Thr Asn Ala Glu Asp Gly Lys Ile Leu His Glu
       450                 455                 460
   Met Gly Gly His Leu Ser Glu Val Leu Leu Leu Ser Leu Val Val Leu
   465                 470                 475                 480
   Ser Asp Phe Ala Pro Glu Thr Ala Ser Val Met Tyr Leu Ile Leu His
                   485                 490                 495
   Phe Ser Ile Pro Gln Ser His Val Asp Val Met Asp Cys Asp Lys Thr
               500                 505                 510
   Gln Leu Asn Leu Thr Val Glu Leu Thr Thr Ala Glu Val Ile Pro Gly
           515                 520                 525
   Ser Val Trp Asn Leu Gly Lys Tyr Val Cys Ile Arg Pro Asn Trp Trp
       530                 535                 540
   Pro Tyr Glu Thr Thr Val Val Leu Ala Phe Glu Glu Val Ser Gln Val
   545                 550                 555                 560
   Val Lys Leu Val Leu Arg Ala Leu Arg Asp Leu Thr Arg Ile Trp Asn
                   565                 570                 575
   Ala Ala Thr Thr Thr Ala Phe Leu Val Cys Leu Val Lys Ile Val Arg
               580                 585                 590
   Gly Gln Met Val Gln Gly Ile Leu Trp Leu Leu Leu Ile Thr Gly Val
           595                 600                 605
   Gln Gly His Leu Asp Cys Lys Pro Glu Phe Ser Tyr Ala Ile Ala Lys
       610                 615                 620
   Asp Glu Arg Ile Gly Gln Leu Gly Ala Glu Gly Leu Thr Thr Thr Trp
   625                 630                 635                 640
   Lys Glu Tyr Ser Pro Gly Met Lys Leu Glu Asp Thr Met Val Ile Ala
                   645                 650                 655
   Trp Cys Glu Asp Gly Lys Leu Met Tyr Leu Gln Arg Cys Thr Arg Glu
               660                 665                 670
   Thr Arg Tyr Leu Ala Ile Leu His Thr Arg Ala Leu Pro Thr Ser Val
           675                 680                 685
   Val Phe Lys Lys Leu Phe Asp Gly Arg Lys Gln Glu Asp Val Val Glu
       690                 695                 700
   Met Asn Asp Asn Phe Glu Phe Gly Leu Cys Pro Cys Asp Ala Lys Pro
   705                 710                 715                 720
   Ile Val Arg Gly Lys Phe Asn Thr Thr Leu Leu Asn Gly Pro Ala Phe
                   725                 730                 735
   Gln Met Val Cys Pro Ile Gly Trp Thr Gly Thr Val Ser Cys Thr Ser
               740                 745                 750
   Phe Asn Met Asp Thr Leu Ala Thr Thr Val Val Arg Thr Tyr Arg Arg
           755                 760                 765
   Ser Lys Pro Phe Pro His Arg Gln Gly Cys Ile Thr Gln Lys Asn Leu
       770                 775                 780
   Gly Glu Asp Leu His Asn Cys Ile Leu Gly Gly Asn Trp Thr Cys Val
   785                 790                 795                 800
   Pro Gly Asp Gln Leu Leu Tyr Lys Gly Gly Ser Ile Glu Ser Cys Lys
                   805                 810                 815
   Trp Cys Gly Tyr Gln Phe Lys Glu Ser Glu Gly Leu Pro His Tyr Pro
               820                 825                 830
   Ile Gly Lys Cys Lys Leu Glu Asn Glu Thr Gly Tyr Arg Leu Val Asp
           835                 840                 845
   Ser Thr Ser Cys Asn Arg Glu Gly Val Ala Ile Val Pro Gln Gly Thr
       850                 855                 860
   Leu Lys Cys Lys Ile Gly Lys Thr Thr Val Gln Val Ile Ala Met Asp
   865                 870                 875                 880
   Thr Lys Leu Gly Pro Met Pro Cys Arg Pro Tyr Glu Ile Ile Ser Ser
                   885                 890                 895
   Glu Gly Pro Val Glu Lys Thr Ala Cys Thr Phe Asn Tyr Thr Lys Thr
               900                 905                 910
   Leu Lys Asn Lys Tyr Phe Glu Pro Arg Asp Ser Tyr Phe Gln Gln Tyr
           915                 920                 925
   Met Leu Lys Gly Glu Tyr Gln Tyr Trp Phe Asp Leu Glu Val Thr Asp
       930                 935                 940
   His His Arg Asp Tyr Phe Ala Glu Ser Ile Leu Val Val Val Val Ala
   945                 950                 955                 960
   Leu Leu Gly Gly Arg Tyr Val Leu Trp Leu Leu Val Thr Tyr Met Val
   965 970 975
   Leu Ser Glu Gln Lys Ala Leu Gly Ile Gln Tyr Gly Ser Gly Glu Val
   980 985 990
   Val Met Met Gly Asn Leu Leu Thr His Asn Asn Ile Glu Val Val Thr
   995 1000 1005
   Tyr Phe Leu Leu Leu Tyr Leu Leu Leu Arg Glu Glu Ser Val Lys
   1010 1015 1020
   Lys Trp Val Leu Leu Leu Tyr His Ile Leu Val Val His Pro Ile
   1025 1030 1035
   Lys Ser Val Ile Val Ile Leu Leu Met Ile Gly Asp Val Val Lys
   1040 1045 1050
   Ala Asp Ser Gly Gly Gln Glu Tyr Leu Gly Lys Ile Asp Leu Cys
   1055 1060 1065
   Phe Thr Thr Val Val Leu Ile Val Ile Gly Leu Ile Ile Ala Arg
   1070 1075 1080
   Arg Asp Pro Thr Ile Val Pro Leu Val Thr Ile Met Ala Ala Leu
   1085 1090 1095
   Arg Val Thr Glu Leu Thr His Gln Pro Gly Val Asp Ile Ala Val
   1100 1105 1110
   Ala Val Met Thr Ile Thr Leu Leu Met Val Ser Tyr Val Thr Asp
   1115 1120 1125
   Tyr Phe Arg Tyr Lys Lys Trp Leu Gln Cys Ile Leu Ser Leu Val
   1130 1135 1140
   Ser Ala Val Phe Leu Ile Arg Ser Leu Ile Tyr Leu Gly Arg Ile
   1145 1150 1155
   Glu Met Pro Glu Val Thr Ile Pro Asn Trp Arg Pro Leu Thr Leu
   1160 1165 1170
   Ile Leu Leu Tyr Leu Ile Ser Thr Thr Ile Val Thr Arg Trp Lys
   1175 1180 1185
   Val Asp Val Ala Gly Leu Leu Leu Gln Cys Val Pro Ile Leu Leu
   1190 1195 1200
   Leu Val Thr Thr Leu Trp Ala Asp Phe Leu Thr Leu Ile Leu Ile
   1205 1210 1215
   Leu Pro Thr Tyr Glu Leu Val Lys Leu Tyr Tyr Leu Lys Thr Val
   1220 1225 1230
   Arg Thr Asp Thr Glu Arg Ser Trp Leu Gly Gly Ile Asp Tyr Thr
   1235 1240 1245
   Arg Val Asp Ser Ile Tyr Asp Val Asp Glu Ser Gly Glu Gly Val
   1250 1255 1260
   Tyr Leu Phe Pro Ser Arg Gln Lys Ala Gln Gly Asn Phe Ser Ile
   1265 1270 1275
   Leu Leu Pro Leu Ile Lys Ala Thr Leu Ile Ser Cys Val Ser Ser
   1280 1285 1290
   Lys Trp Gln Leu Ile Tyr Met Ser Tyr Leu Thr Leu Asp Phe Met
   1295 1300 1305
   Tyr Tyr Met His Arg Lys Val Ile Glu Glu Ile Ser Gly Gly Thr
   1310 1315 1320
   Asn Ile Ile Ser Arg Leu Val Ala Ala Leu Ile Glu Leu Asn Trp
   1325 1330 1335
   Ser Met Glu Glu Glu Glu Ser Lys Gly Leu Lys Lys Phe Tyr Leu
   1340 1345 1350
   Leu Ser Gly Arg Leu Arg Asn Leu Ile Ile Lys His Lys Val Arg
   1355 1360 1365
   Asn Glu Thr Val Ala Ser Trp Tyr Gly Glu Glu Glu Val Tyr Gly
   1370 1375 1380
   Met Pro Lys Ile Met Thr Ile Ile Lys Ala Ser Thr Leu Ser Lys
   1385 1390 1395
   Ser Arg His Cys Ile Ile Cys Thr Val Cys Glu Gly Arg Glu Trp
   1400 1405 1410
   Lys Gly Gly Thr Cys Pro Lys Cys Gly Arg His Gly Lys Pro Ile
   1415 1420 1425
   Thr Cys Gly Met Ser Leu Ala Asp Phe Glu Glu Arg His Tyr Lys
   1430 1435 1440
   Arg Ile Phe Ile Arg Glu Gly Asn Phe Glu Gly Met Cys Ser Arg
   1445 1450 1455
   Cys Gln Gly Lys His Arg Arg Phe Glu Met Asp Arg Glu Pro Lys
   1460 1465 1470
   Ser Ala Arg Tyr Cys Ala Glu Cys Asn Arg Leu His Pro Ala Glu
       1475                    1480                    1485
   Glu Gly Asp Phe Trp Ala Glu Ser Ser Met Leu Gly Leu Lys Ile
       1490                    1495                    1500
   Thr Tyr Phe Ala Leu Met Asp Gly Lys Val Tyr Asp Ile Thr Glu
       1505                    1510                    1515
   Trp Ala Gly Cys Gln Arg Val Gly Ile Ser Pro Asp Thr His Arg
       1520                    1525                    1530
   Val Pro Cys His Ile Ser Phe Gly Ser Arg Met Pro Phe Arg Gln
       1535                    1540                    1545
   Glu Tyr Asn Gly Phe Val Gln Tyr Thr Ala Arg Gly Gln Leu Phe
       1550                    1555                    1560
   Leu Arg Asn Leu Pro Val Leu Ala Thr Lys Val Lys Met Leu Met
       1565                    1570                    1575
   Val Gly Asn Leu Gly Glu Glu Ile Gly Asn Leu Glu His Leu Gly
       1580                    1585                    1590
   Trp Ile Leu Arg Gly Pro Ala Val Cys Lys Lys Ile Thr Glu His
       1595                    1600                    1605
   Glu Lys Cys His Ile Asn Ile Leu Asp Lys Leu Thr Ala Phe Phe
       1610                    1615                    1620
   Gly Ile Met Pro Arg Gly Thr Thr Pro Arg Ala Pro Val Arg Phe
       1625                    1630                    1635
   Pro Thr Ser Leu Leu Lys Val Arg Arg Gly Leu Glu Thr Ala Trp
       1640                    1645                    1650
   Ala Tyr Thr His Gln Gly Gly Ile Ser Ser Val Asp His Val Thr
       1655                    1660                    1665
   Ala Gly Lys Asp Leu Leu Val Cys Asp Ser Met Gly Arg Thr Arg
       1670                    1675                    1680
   Val Val Cys Gln Ser Asn Asn Arg Leu Thr Asp Glu Thr Glu Tyr
       1685                    1690                    1695
   Gly Val Lys Thr Asp Ser Gly Cys Pro Asp Gly Ala Arg Cys Tyr
       1700                    1705                    1710
   Val Leu Asn Pro Glu Ala Val Asn Ile Ser Gly Ser Lys Gly Ala
       1715                    1720                    1725
   Val Val His Leu Gln Lys Thr Gly Gly Glu Phe Thr Cys Val Thr
       1730                1735                1740
   Ala Ser Gly Thr Pro Ala Phe Phe Asp Leu Lys Asn Leu Lys Gly
       1745                1750                1755
   Trp Ser Gly Leu Pro Ile Phe Glu Ala Ser Ser Gly Arg Val Val
       1760                1765                1770
   Gly Arg Val Lys Val Gly Lys Asn Glu Glu Ser Lys Pro Thr Lys
       1775                1780                1785
   Ile Met Ser Gly Ile Gln Thr Val Ser Lys Asn Arg Ala Asp Leu
       1790                1795                1800
   Thr Glu Met Val Lys Lys Ile Thr Ser Met Asn Arg Gly Asp Phe
       1805                1810                1815
   Lys Gln Ile Thr Leu Ala Thr Gly Ala Gly Lys Thr Thr Glu Leu
       1820                1825                1830
   Pro Lys Ala Val Ile Glu Glu Ile Gly Arg His Lys Arg Val Leu
       1835                1840                1845
   Val Leu Ile Pro Leu Arg Ala Ala Ala Glu Ser Val Tyr Gln Tyr
       1850                1855                1860
   Met Arg Leu Lys His Pro Ser Ile Ser Phe Asn Leu Arg Ile Gly
       1865                1870                1875
   Asp Met Lys Glu Gly Asp Met Ala Thr Gly Ile Thr Tyr Ala Ser
       1880                1885                1890
   Tyr Gly Tyr Phe Cys Gln Met Pro Gln Pro Lys Leu Arg Ala Ala
       1895                1900                1905
   Met Val Glu Tyr Ser Tyr Ile Phe Leu Asp Glu Tyr His Cys Ala
       1910                1915                1920
   Thr Pro Glu Gln Leu Ala Ile Ile Gly Lys Ile His Arg Phe Ser
       1925                1930                1935
   Glu Ser Ile Arg Val Val Ala Met Thr Ala Thr Pro Ala Gly Ser
       1940                1945                1950
   Val Thr Thr Thr Gly Gln Lys His Pro Ile Glu Glu Phe Ile Ala
       1955                1960                1965
   Pro Glu Val Met Lys Gly Glu Asp Leu Gly Ser Gln Phe Leu Asp
   1970 1975 1980
   Ile Ala Gly Leu Lys Ile Pro Val Asp Glu Met Lys Gly Asn Met
   1985 1990 1995
   Leu Val Phe Val Pro Thr Arg Asn Met Ala Val Glu Val Ala Lys
   2000 2005 2010
   Lys Leu Lys Ala Lys Gly Tyr Asn Ser Gly Tyr Tyr Tyr Ser Gly
   2015 2020 2025
   Glu Asp Pro Ala Asn Leu Arg Val Val Thr Ser Gln Ser Pro Tyr
   2030 2035 2040
   Val Ile Val Ala Thr Asn Ala Ile Glu Ser Gly Val Thr Leu Pro
   2045 2050 2055
   Asp Leu Asp Thr Val Ile Asp Thr Gly Leu Lys Cys Glu Lys Arg
   2060 2065 2070
   Val Arg Val Ser Ser Lys Ile Pro Phe Ile Val Thr Gly Leu Lys
   2075 2080 2085
   Arg Met Ala Val Thr Val Gly Glu Gln Ala Gln Arg Arg Gly Arg
   2090 2095 2100
   Val Gly Arg Val Lys Pro Gly Arg Tyr Tyr Arg Ser Gln Glu Thr
   2105 2110 2115
   Ala Thr Gly Ser Lys Asp Tyr His Tyr Asp Leu Leu Gln Ala Gln
   2120 2125 2130
   Arg Tyr Gly Ile Glu Asp Gly Ile Asn Val Thr Lys Ser Phe Arg
   2135 2140 2145
   Glu Met Asn Tyr Asp Trp Ser Leu Tyr Glu Glu Asp Ser Leu Leu
   2150 2155 2160
   Ile Thr Gln Leu Glu Ile Leu Asn Asn Leu Leu Ile Ser Glu Asp
   2165 2170 2175
   Leu Pro Ala Ala Val Lys Asn Ile Met Ala Arg Thr Asp His Pro
   2180 2185 2190
   Glu Pro Ile Gln Leu Ala Tyr Asn Ser Tyr Glu Val Gln Val Pro
   2195 2200 2205
   Val Leu Phe Pro Lys Ile Arg Asn Gly Glu Val Thr Asp Thr Tyr
   2210 2215 2220
   Glu Asn Tyr Ser Phe Leu Asn Ala Arg Lys Leu Gly Glu Asp Val
   2225 2230 2235
   Pro Val Tyr Ile Tyr Ala Thr Glu Asp Glu Asp Leu Ala Val Asp
   2240 2245 2250
   Leu Leu Gly Leu Asp Trp Pro Asp Pro Gly Asn Gln Gln Val Val
   2255 2260 2265
   Glu Thr Gly Lys Ala Leu Lys Gln Val Thr Gly Leu Ser Ser Ala
   2270 2275 2280
   Glu Asn Ala Leu Leu Val Ala Leu Phe Gly Tyr Val Gly Tyr Gln
   2285 2290 2295
   Ala Leu Ser Lys Arg His Val Pro Met Ile Thr Asp Ile Tyr Thr
   2300 2305 2310
   Ile Glu Asp Gln Arg Leu Glu Asp Thr Thr His Leu Gln Tyr Ala
   2315 2320 2325
   Pro Asn Ala Ile Lys Thr Asp Gly Thr Glu Thr Glu Leu Lys Glu
   2330 2335 2340
   Leu Ala Ser Gly Asp Val Glu Lys Ile Met Gly Ala Ile Ser Asp
   2345 2350 2355
   Tyr Ala Ala Gly Gly Leu Glu Phe Val Lys Ser Gln Ala Glu Lys
   2360 2365 2370
   Ile Lys Thr Ala Pro Leu Phe Lys Glu Asn Ala Glu Ala Ala Lys
   2375 2380 2385
   Gly Tyr Val Gln Lys Phe Ile Asp Ser Leu Ile Glu Asn Lys Glu
   2390 2395 2400
   Glu Ile Ile Arg Tyr Gly Leu Trp Gly Thr His Thr Ala Leu Tyr
   2405 2410 2415
   Lys Ser Ile Ala Ala Arg Leu Gly His Glu Thr Ala Phe Ala Thr
   2420 2425 2430
   Leu Val Leu Lys Trp Leu Ala Phe Gly Gly Glu Ser Val Ser Asp
   2435 2440 2445
   His Val Lys Gln Ala Ala Val Asp Leu Val Val Tyr Tyr Val Met
   2450 2455 2460
   Asn Lys Pro Ser Phe Pro Gly Asp Ser Glu Thr Gln Gln Glu Gly
   2465 2470 2475
   Arg Arg Phe Val Ala Ser Leu Phe Ile Ser Ala Leu Ala Thr Tyr
   2480 2485 2490
   Thr Tyr Lys Thr Trp Asn Tyr His Asn Leu Ser Lys Val Val Glu
   2495 2500 2505
   Pro Ala Leu Ala Tyr Leu Pro Tyr Ala Thr Ser Ala Leu Lys Met
   2510 2515 2520
   Phe Thr Pro Thr Arg Leu Glu Ser Val Val Ile Leu Ser Thr Thr
   2525 2530 2535
   Ile Tyr Lys Thr Tyr Leu Ser Ile Arg Lys Gly Lys Ser Asp Gly
   2540 2545 2550
   Leu Leu Gly Thr Gly Ile Ser Ala Ala Met Glu Ile Leu Ser Gln
   2555 2560 2565
   Asn Pro Val Ser Val Gly Ile Ser Val Met Leu Gly Val Gly Ala
   2570 2575 2580
   Ile Ala Ala His Asn Ala Ile Glu Ser Ser Glu Gln Lys Arg Thr
   2585 2590 2595
   Leu Leu Met Lys Val Phe Val Lys Asn Phe Leu Asp Gln Ala Ala
   2600 2605 2610
   Thr Asp Glu Leu Val Lys Glu Asn Pro Glu Lys Ile Ile Met Ala
   2615 2620 2625
   Leu Phe Glu Ala Val Gln Thr Ile Gly Asn Pro Leu Arg Leu Ile
   2630 2635 2640
   Tyr His Leu Tyr Gly Val Tyr Tyr Lys Gly Trp Glu Ala Lys Glu
   2645 2650 2655
   Leu Ser Glu Arg Thr Ala Gly Arg Asn Leu Phe Thr Leu Ile Met
   2660 2665 2670
   Phe Glu Ala Phe Glu Leu Leu Gly Met Asp Ser Gln Gly Lys Ile
   2675 2680 2685
   Arg Asn Leu Ser Gly Asn Tyr Ile Leu Asp Leu Ile Tyr Gly Leu
   2690 2695 2700
   His Lys Gln Ile Asn Arg Gly Leu Lys Lys Met Val Leu Gly Trp
   2705 2710 2715
   Ala Pro Ala Pro Phe Ser Cys Asp Trp Thr Pro Ser Asp Glu Arg
   2720 2725 2730
   Ile Arg Leu Pro Thr Asp Asn Tyr Leu Arg Val Glu Thr Arg Cys
   2735 2740 2745
   Pro Cys Gly Tyr Glu Met Lys Ala Phe Lys Asn Val Gly Gly Lys
   2750 2755 2760
   Leu Thr Lys Val Glu Glu Ser Gly Pro Phe Leu Cys Arg Asn Arg
   2765 2770 2775
   Pro Gly Arg Gly Pro Val Asn Tyr Arg Val Thr Lys Tyr Tyr Asp
   2780 2785 2790
   Asp Asn Leu Arg Glu Ile Lys Pro Val Ala Lys Leu Glu Gly Gln
   2795 2800 2805
   Val Glu His Tyr Tyr Lys Gly Val Thr Ala Lys Ile Asp Tyr Ser
   2810 2815 2820
   Lys Gly Lys Met Leu Leu Ala Thr Asp Lys Trp Glu Val Glu His
   2825 2830 2835
   Gly Val Ile Thr Arg Leu Ala Lys Arg Tyr Thr Gly Val Gly Phe
   2840 2845 2850
   Asn Gly Ala Tyr Leu Gly Asp Glu Pro Asn His Arg Ala Leu Val
   2855 2860 2865
   Glu Arg Asp Cys Ala Thr Ile Thr Lys Asn Thr Val Gln Phe Leu
   2870 2875 2880
   Lys Met Lys Lys Gly Cys Ala Phe Thr Tyr Asp Leu Thr Ile Ser
   2885 2890 2895
   Asn Leu Thr Arg Leu Ile Glu Leu Val His Arg Asn Asn Leu Glu
   2900 2905 2910
   Glu Lys Glu Ile Pro Thr Ala Thr Val Thr Thr Trp Leu Ala Tyr
   2915 2920 2925
   Thr Phe Val Asn Glu Asp Val Gly Thr Ile Lys Pro Val Leu Gly
   2930 2935 2940
   Glu Arg Val Ile Pro Asp Pro Val Val Asp Ile Asn Leu Gln Pro
   2945 2950 2955
   Glu Val Gln Val Asp Thr Ser Glu Val Gly Ile Thr Ile Ile Gly
   2960 2965 2970
   Arg Glu Thr Leu Met Thr Thr Gly Val Thr Pro Val Leu Glu Lys
   2975 2980 2985
   Val Glu Pro Asp Ala Ser Asp Asn Gln Asn Ser Val Lys Ile Gly
   2990 2995 3000
   Leu Asp Glu Gly Asn Tyr Pro Gly Pro Gly Ile Gln Thr His Thr
   3005 3010 3015
   Leu Thr Glu Glu Ile His Asn Arg Asp Ala Arg Pro Phe Ile Met
   3020 3025 3030
   Ile Leu Gly Ser Arg Asn Ser Ile Ser Asn Arg Ala Lys Thr Ala
   3035 3040 3045
   Arg Asn Ile Asn Leu Tyr Thr Gly Asn Asp Pro Arg Glu Ile Arg
   3050 3055 3060
   Asp Leu Met Ala Ala Gly Arg Met Leu Val Val Ala Leu Arg Asp
   3065 3070 3075
   Val Asp Pro Glu Leu Ser Glu Met Val Asp Phe Lys Gly Thr Phe
   3080 3085 3090
   Leu Asp Arg Glu Ala Leu Glu Ala Leu Ser Leu Gly Gln Pro Lys
   3095 3100 3105
   Pro Lys Gln Val Thr Lys Glu Ala Val Arg Asn Leu Ile Glu Gln
   3110 3115 3120
   Lys Lys Asp Val Glu Ile Pro Asn Trp Phe Ala Ser Asp Asp Pro
   3125 3130 3135
   Val Phe Leu Glu Val Ala Leu Lys Asn Asp Lys Tyr Tyr Leu Val
   3140 3145 3150
   Gly Asp Val Gly Glu Leu Lys Asp Gln Ala Lys Ala Leu Gly Ala
   3155 3160 3165
   Thr Asp Gln Thr Arg Ile Ile Lys Glu Val Gly Ser Arg Thr Tyr
   3170 3175 3180
   Ala Met Lys Leu Ser Ser Trp Phe Leu Lys Ala Ser Asn Lys Gln
   3185 3190 3195
   Met Ser Leu Thr Pro Leu Phe Glu Glu Leu Leu Leu Arg Cys Pro
   3200 3205 3210
   Pro Ala Thr Lys Ser Asn Lys Gly His Met Ala Ser Ala Tyr Gln
   3215 3220 3225
   Leu Ala Gln Gly Asn Trp Glu Pro Leu Gly Cys Gly Val His Leu
   3230 3235 3240
   Gly Thr Ile Pro Ala Arg Arg Val Lys Ile His Pro Tyr Glu Ala
   3245 3250 3255
   Tyr Leu Lys Leu Lys Asp Phe Ile Glu Glu Glu Glu Lys Lys Pro
   3260 3265 3270
   Arg Val Lys Asp Thr Val Ile Arg Glu His Asn Lys Trp Ile Leu
   3275 3280 3285
   Lys Lys Ile Arg Phe Gln Gly Asn Leu Asn Thr Lys Lys Met Leu
   3290 3295 3300
   Asn Pro Gly Lys Leu Ser Glu Gln Leu Asp Arg Glu Gly Arg Lys
   3305 3310 3315
   Arg Asn Ile Tyr Asn His Gln Ile Gly Thr Ile Met Ser Ser Ala
   3320 3325 3330
   Gly Ile Arg Leu Glu Lys Leu Pro Ile Val Arg Ala Gln Thr Asp
   3335 3340 3345
   Thr Lys Thr Phe His Glu Ala Ile Arg Asp Lys Ile Asp Lys Ser
   3350 3355 3360
   Glu Asn Arg Gln Asn Pro Glu Leu His Asn Lys Leu Leu Glu Ile
   3365 3370 3375
   Phe His Thr Ile Ala Gln Pro Thr Leu Lys His Thr Tyr Gly Glu
   3380 3385 3390
   Val Thr Trp Glu Gln Leu Glu Ala Gly Val Asn Arg Lys Gly Ala
   3395 3400 3405
   Ala Gly Phe Leu Glu Lys Lys Asn Ile Gly Glu Val Leu Asp Ser
   3410 3415 3420
   Glu Lys His Leu Val Glu Gln Leu Val Arg Asp Leu Lys Ala Gly
   3425 3430 3435
   Arg Lys Ile Lys Tyr Tyr Glu Thr Ala Ile Pro Lys Asn Glu Lys
   3440 3445 3450
   Arg Asp Val Ser Asp Asp Trp Gln Ala Gly Asp Leu Val Val Glu
   3455 3460 3465
   Lys Arg Pro Arg Val Ile Gln Tyr Pro Glu Ala Lys Thr Arg Leu
   3470 3475 3480
   Ala Ile Thr Lys Val Met Tyr Asn Trp Val Lys Gln Gln Pro Val
       3485                3490                3495
   Val Ile Pro Gly Tyr Glu Gly Lys Thr Pro Leu Phe Asn Ile Phe
       3500                3505                3510
   Asp Lys Val Arg Lys Glu Trp Asp Ser Phe Asn Glu Pro Val Ala
       3515                3520                3525
   Val Ser Phe Asp Thr Lys Ala Trp Asp Thr Gln Val Thr Ser Lys
       3530                3535                3540
   Asp Leu Gln Leu Ile Gly Glu Ile Gln Lys Tyr Tyr Tyr Lys Lys
       3545                3550                3555
   Glu Trp His Lys Phe Ile Asp Thr Ile Thr Asp His Met Thr Glu
       3560                3565                3570
   Val Pro Val Ile Thr Ala Asp Gly Glu Val Tyr Ile Arg Asn Gly
       3575                3580                3585
   Gln Arg Gly Ser Gly Gln Pro Asp Thr Ser Ala Gly Asn Ser Met
       3590                3595                3600
   Leu Asn Val Leu Thr Met Met Tyr Gly Phe Cys Glu Ser Thr Gly
       3605                3610                3615
   Val Pro Tyr Lys Ser Phe Asn Arg Val Ala Arg Ile His Val Cys
       3620                3625                3630
   Gly Asp Asp Gly Phe Leu Ile Thr Glu Lys Gly Leu Gly Leu Lys
       3635                3640                3645
   Phe Ala Asn Lys Gly Met Gln Ile Leu His Glu Ala Gly Lys Pro
       3650                3655                3660
   Gln Lys Ile Thr Glu Gly Glu Lys Met Lys Val Ala Tyr Arg Phe
       3665                3670                3675
   Glu Asp Ile Glu Phe Cys Ser His Thr Pro Val Pro Val Arg Trp
       3680                3685                3690
   Ser Asp Asn Thr Ser Ser His Met Ala Gly Arg Asp Thr Ala Val
       3695                3700                3705
   Ile Leu Ser Lys Met Ala Thr Arg Leu Asp Ser Ser Gly Glu Arg
       3710                3715                3720
   Gly Thr Thr Ala Tyr Glu Lys Ala Val Ala Phe Ser Phe Leu Leu
       3725                3730                3735
   Met Tyr Ser Trp Asn Pro Leu Val Arg Arg Ile Cys Leu Leu Val
   3740 3745 3750
   Leu Ser Gln Gln Pro Glu Thr Asp Pro Ser Lys His Ala Thr Tyr
   3755 3760 3765
   Tyr Tyr Lys Gly Asp Pro Ile Gly Ala Tyr Lys Asp Val Ile Gly
   3770 3775 3780
   Arg Asn Leu Ser Glu Leu Lys Arg Thr Gly Phe Glu Lys Leu Ala
   3785 3790 3795
   Asn Leu Asn Leu Ser Leu Ser Thr Leu Gly Val Trp Thr Lys His
   3800 3805 3810
   Thr Ser Lys Arg Ile Ile Gln Asp Cys Val Ala Ile Gly Lys Glu
   3815 3820 3825
   Glu Gly Asn Trp Leu Val Lys Pro Asp Arg Leu Ile Ser Ser Lys
   3830 3835 3840
   Thr Gly His Leu Tyr Ile Pro Asp Lys Gly Phe Thr Leu Gln Gly
   3845 3850 3855
   Lys His Tyr Glu Gln Leu Gln Leu Arg Thr Glu Thr Asn Pro Val
   3860 3865 3870
   Met Gly Val Gly Thr Glu Arg Tyr Lys Leu Gly Pro Ile Val Asn
   3875 3880 3885
   Leu Leu Leu Arg Arg Leu Lys Ile Leu Leu Met Thr Ala Val Gly
   3890 3895 3900
   Val Ser Ser
   3905
<210> 2
   <211> 18
   <212> DNA
   <213> Artificial
<220>
   <223> Primer - Flanks 5' end of p15aDI cloning site for mutant fragments
<400> 2
   gaggccgtta acatatca 18
<210> 3
   <211> 27
   <212> DNA
   <213> Artificial
<220>
   <223> Primer - Flanks 3' end of p15aDI cloning site for mutant fragments
<400> 3
   cctaaatcac tttgaccctg ttgctgt 27
<210> 4
   <211> 30
   <212> DNA
   <213> Artificial
<220>
   <223> Primer - 5' primer for introducing I1841A mutation
<400> 4
   gaggcagggc gccacaagag agtattagtt 30
<210> 5
   <211> 32
   <212> DNA
   <213> Artificial
<220>
   <223> 3' primer for introducing I1841A mutation
<400> 5
   cttgtggcgc cctgcctcct ctataactgc tt 32
<210> 6
   <211> 30
   <212> DNA
   <213> Artificial
<220>
   <223> 5' primer for introducing R1843A mutation
<400> 6
   gagataggcg cccacaagag agtattagtt 30
<210> 7
   <211> 27
   <212> DNA
   <213> Artificial
<220>
   <223> 3' primer for introducing R1843A mutation
<400> 7
   cttgtgggcg cctatctcct ctataac 27
<210> 8
   <211> 32
   <212> DNA
   <213> Artificial
<220>
   <223> 5' primer for introducing K1845A mutation
<400> 8
   atagggcgcc acgcgagagt attagttctt at 32
<210> 9
   <211> 30
   <212> DNA
   <213> Artificial
<220>
   <223> 3' primer for introducing K1845A mutation
<400> 9
   tctcgcgtgg cgccctatct cctctataac 30
<210> 10
   <211> 34
   <212> DNA
   <213> Artificial
<220>
   <223> 5' primer for introducing K1867A mutation
<400> 10
   ttggctcacc catcgatctc ttttaaccta agga 34
<210> 11
   <211> 37
   <212> DNA
   <213> Artificial
<220>
   <223> 3' primer for introducing K1867A mutation
<400> 11
   agagatcgat gggtgagcca atctcatata ctggtag 37
<210> 12
   <211> 31
   <212> DNA
   <213> Artificial
<220>
   <223> 5' primer for introducing H1868A mutation
<400> 12
   aaagctccat cgatctcttt taacctaagg a 31
<210> 13
   <211> 34
   <212> DNA
   <213> Artificial
<220>
   <223> 3' primer for introducing H1868A mutation
<400> 13
   agagatcgat ggagctttca atctcatata ctgg 34
<210> 14
   <211> 34
   <212> DNA
   <213> Artificial
<220>
   <223> 5' primer for introducing P1869A mutation
<400> 14
   cacgcgagca taagctttaa cctaaggata gggg 34
<210> 15
   <211> 36
   <212> DNA
   <213> Artificial
<220>
   <223> 3' primer for introducing P1869A mutation
<400> 15
   ttaaagctta tgctcgcgtg tttcaatctc atatac 36
<210> 16
   <211> 32
   <212> DNA
   <213> Artificial
<220>
   <223> 5' primer for introducing E1939A mutation
<400> 16
   ccatcgattt tcagcgagta taagggttgt cg 32
<210> 17
   <211> 32
   <212> DNA
   <213> Artificial
<220>
   <223> 3' primer for introducing E1939A mutation
<400> 17
   ctcgctgaaa atcgatggat cttcccgata at 32
<210> 18
   <211> 42
   <212> DNA
   <213> Artificial
<220>
   <223> 5' primer for introducing R1942A mutation
<400> 18
   ccatcgattt tcagagagta tagcggttgt cgccatgact gc 42
<210> 19
   <211> 41
   <212> DNA
   <213> Artificial
<220>
   <223> 3' primer for introducing R1942A mutation
<400> 19
   accgctatac tctctgaaaa tcgatggatc ttcccgataa t 41
<210> 20
   <211> 166
   <212> PRT
   <213> Artificial
<220>
   <223> Fragment of NS3 domain of BVDV
<400> 20
   Ser Lys Asn Arg Ala Asp Leu Thr Glu Met Val Lys Lys Ile Thr Ser
   1               5                   10                  15
   Met Asn Arg Gly Asp Phe Lys Gln Ile Thr Leu Ala Thr Gly Ala Gly
               20                  25                  30
   Lys Thr Thr Glu Leu Pro Lys Ala Val Ile Glu Glu Ile Gly Arg His
           35                  40                  45
   Lys Arg Val Leu Val Leu Ile Pro Leu Arg Ala Ala Ala Glu Ser Val
       50                  55                  60
   Tyr Gln Tyr Met Arg Leu Lys His Pro Ser Ile Ser Phe Asn Leu Arg
   65                  70                  75                  80
   Ile Gly Asp Met Lys Glu Gly Asp Met Ala Thr Gly Ile Thr Tyr Ala
                   85                  90                  95
   Ser Tyr Gly Tyr Phe Cys Gln Met Pro Gln Pro Lys Leu Arg Ala Ala
               100                 105                 110
   Met Val Glu Tyr Ser Tyr Ile Phe Leu Asp Glu Tyr His Cys Ala Thr
           115                 120                 125
   Pro Glu Gln Leu Ala Ile Ile Gly Lys Ile His Arg Phe Ser Glu Ser
       130                 135                 140
   Ile Arg Val Val Ala Met Thr Ala Thr Pro Ala Gly Ser Val Thr Thr
   145                 150                 155                 160
   Thr Gly Gln Lys His Pro
                   165
<210> 21
   <211> 145
   <212> PRT
   <213> Artificial
<220>
   <223> Fragment of NS3 domain of HCV
<400> 21
   Pro Pro Ala Val Pro Gln Thr Phe Gln Val Ala His Leu His Ala Pro
   1               5                   10                  15
   Thr Gly Ser Gly Lys Ser Thr Lys Val Pro Ala Ala Tyr Ala Ala Gln
               20                  25                  30
   Gly Tyr Lys Val Leu Val Leu Asn Pro Ser Val Ala Ala Thr Leu Gly
           35                  40                  45
   Phe Gly Val Tyr Met Ser Lys Ala His Gly Ile Asp Pro Asn Ile Arg
       50                  55                  60
   Thr Gly Val Arg Ala Ile Thr Thr Gly Gly Pro Ile Thr Tyr Ser Thr
   65                  70                  75                  80
   Tyr Gly Lys Phe Leu Ala Asp Gly Gly Cys Ser Gly Gly Ala Tyr Asp
                   85                  90                  95
   Ile Ile Ile Cys Asp Glu Cys His Ser Thr Asp Ser Thr Ser Ile Leu
               100                 105                 110
   Gly Ile Gly Thr Val Leu Asp Gln Ala Glu Thr Ala Gly Ala Arg Leu
           115                 120                 125
   Val Val Leu Ala Thr Ala Thr Pro Pro Gly Ser Ile Thr Val Pro His
       130                 135                 140
   Pro
   145

## Description

### Background Of The Invention

Bovine viral diarrhea virus (BVD virus, or BVDV) is a small RNA virus of the genus Pestivirus, and family Flaviviridae. It is closely related to viruses which are the causative agents of border disease in sheep and classical swine fever in pigs. Disease caused by BVDV is widespread, and can be economically devastating. BVDV infection can result in breeding problems in cattle, and can cause abortions or premature births. BVDV is capable of crossing the placenta of pregnant cattle, and may result in the birth of persistently infected (PI) calves which are immunotolerant to the virus and persistently viremic for the rest of their lives. (Malmquist, J. Am. Vet. Med. Assoc. 152:763-768 (1968); Ross, et al., J. Am. Vet. Med. Assoc. 188:618-619 (1986)). Infected cattle can also exhibit "mucosal disease", characterized by elevated temperature, diarrhea, coughing and ulcerations of the alimentary mucosa (Olafson, et al., Cornell Vet. 36:205-213 (1946); Ramsey, et al., North Am. Vet. 34:629-633 (1953)). These persistently infected animals provide a source for dissemination of virus within the herd for further outbreaks of mucosal disease (Liess, et al., Dtsch. Tieraerztl. Wschr. 81:481-487 (1974)) and are highly predisposed to infection with microorganisms responsible for causing enteric diseases or pneumonia (Barber, et al., Vet. Rec. 117:459-464 (1985)).

BVD viruses are classified into one of two biotypes. Those of the "cp" biotype induce a cytopathic effect on cultured cells, whereas viruses of the "ncp" biotype do not (Gillespie, et al., Cornell Vet. 50:73-79 (1960)). In addition, two major genotypes (type 1 and 2) are recognized, both of which have been shown to cause a variety of clinical syndromes (Pellerin, et al., Virology 203:260-268 (1994); Ridpath, et al., Virology 205:66-74(1994)). BVD virions are 40 to 60 nm in diameter. The nucleocapsid of BVDV consists of a single molecule of RNA and the capsid protein C. The nucleocapsid is surrounded by a lipid membrane with two glycoproteins anchored in it, E1 and E2. A third glycoprotein, E^{rns}, is loosely associated to the envelope. The genome of BVDV is approximately 12.5 kb in length, and contains a single open reading frame located between the 5' and 3' non-translated regions (NTRs) (Collett, et al., Virology 165:191-199 (1988)). A polyprotein of approximately 438 kD is translated from this open reading frame, and is processed by cellular and viral proteases into at least eleven viral structural and nonstructural (NS) proteins (Tautz, et al., J. Virol. 71:5415-5422 (1997); Xu, et al., J. Virol. 71:5312-5322 (1997); Elbers, et al., J. Virol. 70:4131-4135 (1996); and Wiskerchen, et al., Virology 184:341-350 (1991)). The genomic order of BVDV is p20/N^{pro}, p14/C, gp48/E^{rns}, gp25/E1, gp53/E2, p54/NS2, p80/NS3, p10/NS4A, p32/NS4B, p58/NS5A and p75/NS5B. P20/N^{pro} (Stark, et al., J. Virol. 67:7088-7093 (1993); Wiskerchen, et al., Virol. 65:4508-4514 (1991)) is a cis-acting, papain-like protease that cleaves itself from the rest of the synthesized polyprotein. The capsid protein (C), also referred to as p14, is a basic protein, and functions in packaging of the genomic RNA and formation of the enveloped virion. P14/C is conserved across different pestiviruses. The three envelope proteins, gp48/E^{rns}, gp25/E1 and gp53/E2, are heavily glycosylated. E^{rns} forms homodimers, covalently linked by disulfides. The absence of a hydrophobic membrane anchor region suggests that E^{rns} is loosely associated with the envelope. E^{rns} induces high antibody titers in infected cattle, but the antisera has limited virus-neutralizing activity. E1 is found in virions covalently linked to gp53/E2 via disulfide bonds. E1 contains two hydrophobic regions that serve to anchor the protein in the membrane, and as a signal peptide for initiating translocation. E1 does not induce a significant antibody response in infected cattle, suggesting that it may not be exposed on the virion's surface. Like E1, E2 also has a membrane anchor region at its C-terminus. Unlike E1, however, E2 is very antigenic, being one of the most immunodominant proteins of BVDV. Antibodies binding to E2 can efficiently neutralize a viral infection, suggesting that it may be involved in virus entry. The region of the polyprotein downstream of the structural proteins encodes the nonstructural proteins, and is processed by two viral proteolytic enzymes. The NS2-NS3 junction is cleaved by a zinc-dependent protease encoded within NS2. The C-terminal portion of the BVDV polyprotein encoding NS3, NS4A, NS4B, NS5A and NS5B is processed by a serine protease encoded by the N-terminal domain of NS3. NS3 is another major BVDV immunogen, as infected cattle develop a strong humoral response to it. In contrast, no serum antibodies are found to the other nonstructural proteins in BVDV-infected cattle, and only a weak humoral immune response to NS4A can be detected.

NS3 is found exclusively in cytopathic BVDV isolates, and the region encoding the protein is one of most conserved in the BVDV genome, based on comparisons among BVDV subtypes and other pestiviruses. The C-terminal portion of NS3 encodes a RNA-dependent NTPase/helicase, and based on sequences comparisons of highly conserved helicase amino acid motifs, the BVDV helicase has been classified into the helicase superfamily-2 (SF2). Within this superfamily are similar proteins from the poty-, flavi-, and pestiviruses, including hog cholera (classical swine fever) virus NS3 helicase, and RNA helicases from other flaviviruses, such as West Nile virus, yellow fever virus, hepatitis C virus (HCV) and Japanese encephalitis virus. The molecular structure of the protease and helicase domains of HCV NS3 have been solved (Yao, et al Nat Struct Biol. 4:463-7 (1997); Jin and Peterson, Arch Bioxchem Biophys 323:47-53 (1995)). The protease domain contains the dual β-barrel fold that is commonly seen among members of the chymotrypsin serine protease family. The helicase domain contains two structurally related β-α-β subdomains, and a third subdomain of seven helices and three short β strands, usually referred to as the helicase α-helical subdomain. The nucleoside triphosphate (NTP) and RNA-binding sites, as well as the helicase active site, are surface-exposed, whereas the protease active site is not, and is oriented facing the helicase domain. The protease and helicase domains are covalently connected by a short surface-exposed strand, and interact over a large surface area (∼900 Å²). The helicase active site, however, is oriented away from this area of interaction.

Among the BVDV vaccines currently available are those which contain chemically-inactivated wild-type virus (McClurkin, et al., Arch. Virol. 58:119 (1978); Fernelius, et al., Am. J. Vet. Res. 33:1421-1431 (1972); and Kolar, et al., Am. J. Vet. Res. 33:1415-1420 (1972)). These vaccines typically require the administration of multiple doses, and result in a short-lived immune response; they also do not protect against fetal transmission of the virus (Bolin, Vet. Clin. North Am. Food Anim. Pract. 11:615-625 (1995)). In sheep, a subunit vaccine based on a purified E2 protein has been reported (Bruschke, et al., Vaccine 15:1940-1945 (1997)). Although this vaccine appears to protect fetuses from becoming infected, protection is limited to only the homologous strain of virus, and there is no correlation between antibody titers and protection.

Modified live virus (MLV) BVDV vaccines have been produced using virus that has been attenuated by repeated passaging in bovine or porcine cells (Coggins, et al., Cornell Vet. 51:539 (1961); and Phillips, et al., Am. J. Vet. Res. 36:135 (1975)), or by chemically-induced mutations that confer a temperature-sensitive phenotype on the virus (Lobmann, et al., Am. J. Vet. Res. 45:2498 (1984); and Lobmann, et al., Am. J. Vet. Res. 47:557-561 (1986)). A single dose of a MLV BVDV vaccine has proven sufficient for providing protection from infection, and the duration of immunity can extend for years in vaccinated cattle (Coria, et al., Can. J. Con. Med. 42:239 (1978)). In addition, cross-protection has been reported using MLV vaccines (Martin, et al., In "Proceedings of the Conference of Research Workers in Animal Diseases", 75:183 (1994)). Safety considerations, however- including fetal transmission of the vaccine strain- are a major concern with respect to use of these modified live viral vaccines (Bolin, Vet. Clin. NorthAm. Food Anim. Pract. 11:615-625 (1995)).

Based on the above, it is clear that a need exists for new and more effective vaccines to control the spread of BVDV. Such a vaccine could be invaluable in future national or regional BVDV eradication programs, and could also be combined with other marked cattle vaccines, representing a substantial advance in livestock farming. One such vaccine is a "marked" vaccine. Such a vaccine lacks an antigenic determinant present in wild-type virus. Animals infected with the wild-type virus mount an immune response to the "marker" immunogenic determinant, while non-infected, vaccinated animals do not, as a result of the determinant not being present in the marked vaccine. Through the use of an immunological assay directed against the marker determinant, infected animals could be differentiated from vaccinated, non-infected animals. By culling out the infected animals, the herd could, over time, become BVD-free. In addition to the benefit of removing the threat of BVD disease, certification of a herd as BVD-free has direct freedom of trade economic benefits.

### Summary of the Invention

The present invention is directed to a bovine viral diarrhea virus comprising at least one helicase domain amino acid mutation, wherein the mutation in the helicase domain of NS3 is selected from the group consisting of:
(a)a helicase domain mutation within the IGR loop at amino acid residue 1845;
(b) a helicase domain mutation within the SES loop at amino acid residue 1939 or 1942; or
(c) a helicase domain mutation within the IGR, KHP and SES loops at amino acid residues 1845, 1868 and 1939, respectively;
and results in a loss of recognition by a monoclonal antibody raised against NS3 from wild-type bovine viral diarrhea virus but wherein viral RNA replication and the generation of infectious virus in retained; and wherein the amino acid residues are numbered according to SEQ ID NO: 1.

### Brief Description of the Drawings

These and other features, aspects and advantages of the present invention are illustrated with reference to the following description, appended claims, and accompanying drawings where
FIG. 1 depicts the domains of NS3.
FIG. 2 shows the sequence alignment of BVDV and HCV helicase domains.
FIG. 3 shows an illustration of the molecular model of BVDV helicase.
FIG. 4 shows the location of scanning mutants.
FIG. 5 shows the domain map of the complete full length BVDV precursor and the BVDV subviral replicon structure.

### BRIEF DESCRIPTION OF THE SEQUENCES

SEQ ID NO. 1 is a peptide sequence of a full length, unprocessed polyprotein from bovine viral diarrhea virus. The numbering of the residues in this sequence corresponds to the mutations described herein. For example, a mutation described as "K1845A" means that the Lysine residue at position 1845 of SEQ ID NO. 1 has been replaced by an Alanine residue;
SEQ ID NO. 2 is a sequence of a DNA plasmid fragment that flanks the 5' end of p15aDI cloning site for generating exemplary mutants;
SEQ ID NO. 3 is a sequence of a DNA plasmid fragment that flanks the 3' end of p15aDI cloning site for generating exemplary mutants;
SEQ ID NO. 4 is a sequence of a DNA 5' primer for introducing the I1841A mutation described herein;
SEQ ID NO. 5 is a sequence of a DNA 3' primer for introducing the I1841A mutation described herein;
SEQ ID NO. 6 is a sequence of a DNA 5' primer for introducing the R1843A mutation described herein;
SEQ ID NO. 7 is a sequence of a DNA 3' primer for introducing the R1843A mutation described herein;
SEQ ID NO. 8 is a sequence of a DNA 5' primer for introducing the K1845A mutation described herein;
SEQ ID NO. 9 is a sequence of a DNA 3' primer for introducing the K1845A mutation described herein;
SEQ ID NO. 10 is a sequence of a DNA 5' primer for introducing the K1867A mutation described herein;
SEQ ID NO. 11 is a sequence of a DNA 3' primer for introducing the K1867A mutation described herein;
SEQ ID NO. 12 is a sequence of a DNA 5' primer for introducing the H1868A mutation described herein;
SEQ ID NO. 13 is a sequence of a DNA 3' primer for introducing the H1868A mutation described herein;
SEQ iD NO. 14 is a sequence of a DNA 5' primer for introducing the P1869A mutation described herein;
SEQ ID NO. 15 is a sequence of a DNA 3' primer for introducing the P1869A mutation described herein;
SEQ ID NO. 16 is a sequence of a DNA 5' primer for introducing the E1939A mutation described herein;
SEQ ID NO. 17 is a sequence of a DNA 3' primer for introducing the E1939A mutation described herein;
SEQ ID NO. 18 is a sequence of a DNA 5' primer for introducing the R1942A mutation described herein;
SEQ ID NO. 19 is a sequence of a DNA 3' primer for introducing the R1942A mutation described herein;
SEQ ID NO. 20 is a peptide sequence of domains 1 (helicase) and 2 (NTPase) of the NS3 region of translated BVDV; and
SEQ ID NO. 21 is a peptide sequence of domains 1 (helicase) and 2 (NTPase) of the NS3 region of translated Hepatitis C virus (HCV).

### Definitions

The following definitions may be applied to terms employed in the description of embodiments of the invention. The following definitions supersede any contradictory definitions contained in each individual reference.

Unless otherwise defined herein, scientific and technical terms used in connection with the present invention shall have the meanings that are commonly understood by those of ordinary skill in the art.

The term "amino acid," as used herein, refers to naturally occurring and synthetic amino acids, as well as amino acid analogs and amino acid mimetics that function in a manner similar to the naturally occurring amino acids. Naturally occurring amino acids are those encoded by the genetic code, as well as those amino acids that are later modified, for example, hydroxyproline, carboxyglutamate, and O-phosphoserine. Stereoisomers (e.g., D-amino acids) of the twenty conventional amino acids, unnatural amino acids such as α and α-disubstituted amino acids, N-alkyl amino acids, lactic acid, and other unconventional amino acids may also be suitable components for polypeptides of the present invention. Examples of unconventional amino acids include: 4-hydroxyproline, γ-carboxyglutamate, ε-N,N,N-trimethyllysine, ε-N-acetyllysine, O-phosphoserine, N-acetylserine, N-formylmethionine, 3-methylhistidine, 5-hydroxylysine, σ-N-methylarginine, and other similar amino acids and imino acids. Amino acid analogs refer to compounds that have the same basic chemical structure as a naturally occurring amino acid, i.e., a carbon that is bound to a hydrogen, a carboxyl group, an amino group, and an R group. Exemplary amino acid analogs include, for example, homoserine, norleucine, methionine sulfoxide, and methionine methyl sulfonium. Such analogs have modified R groups (e.g., norleucine) or modified peptide backbones, but retain the same essential chemical structure as a naturally occurring amino acid. Amino acid mimetics refer to chemical compounds that have a structure that is different from the general chemical structure of an amino acid, but that function in a manner similar to a naturally occurring amino acid.

Amino acids may be referred to herein by either their commonly known three letter symbols or by the one-letter symbols recommended by the IUPAC-IUB Biochemical Nomenclature Commission.

| Amino Acids-Single letter codes: | Three letter codes: | Full names |
|---|---|---|
| G: | Gly: | glycine |
| V: | Val: | valine |
| L: | Leu: | leucine |
| A: | Ala: | alanine |
| I: | Ile: | isoleucine |
| S: | Ser: | serine |
| D: | Asp: | aspartic acid |
| K: | Lys: | lysine |
| R: | Arg: | arginine |
| H: | His: | histidine |
| F: | Phe: | phenylalanine |
| Y: | Tyr: | tyrosine |
| T: | Thr: | threonine |
| C: | Cys: | cysteine |
| M: | Met: | methionine |
| E: | Glu: | glutamic acid |
| W: | Trp: | tryptophan |
| P: | Pro: | proline |
| N: | Asn: | asparagine |
| Q: | Gln: | glutamine |
| X: | Xaa | unspecified amino acid |

The term "animal subjects," as used herein, is meant to include any animal that is susceptible to BVDV infections, such as bovine, sheep and swine. By "treating" or "vaccinating" is meant preventing or reducing the risk of infection by a virulent strain of BVDV, ameliorating the symptoms of a BVDV infection, or accelerating the recovery from a BVDV infection.

BVD "viruses", "viral isolates" or "viral strains" as used herein refer to BVD viruses that consist of the viral genome, associated proteins, and other chemical constituents (such as lipids). Ordinarily, the BVD virus has a genome in the form of RNA. RNA can be reverse- transcribed into DNA for use in cloning. Thus, references made herein to nucleic acid and BVD viral sequences encompass both viral RNA sequences and DNA sequences derived from the viral RNA sequences. For convenience, genomic sequences of BVD as depicted in the SEQUENCE LISTING hereinbelow refer to the polypeptide sequence, and primer DNA sequences used in making the exemplary mutations. The corresponding RNA sequence for each is readily apparent to those of skill in the art.

A number of type I and type II BVD viruses are known to those skilled in the art and are available through, e.g., the American Type Culture Collection.

The term "conservative amino acid substitutions," as used herein, are those that generally take place within a family of amino acids that are related in their side chains. In particular, as used herein, a conservative amino acid substitution is one that has no effect on antibody recognition of a given peptide as compared with the wild-type derived peptide. Genetically encoded amino acids are generally divided into four groups: (1) acidic=aspartate, glutamate; (2) basic=lysine, arginine, histidine; (3) non-polar=alanine, valine, leucine, isoleucine, proline, phenylalanine, methionine, tryptophan; and (4) uncharged polar=glycine, asparagine, glutamine, cysteine, serine, threonine, tyrosine. Phenylalanine, tryptophan and tyrosine are also jointly classified as aromatic amino acids.

Accordingly, the term "non-conservative amino acid substitutions," as used herein, are those that are likely to have different properties, particularly with respect to antibody recognition. Thus, a non-conservative amino acid substitution will evoke a differential immune response, such as, for example, loss of recognition by an antibody raised against a wild-type derived peptide.

The term "immunogenic," as used herein, means the capacity of a mutated or wild-type BVD virus in provoking an immune response in an animal against type I or type II BVD viruses, or against both type I and type II BVD viruses. The immune response can be a cellular immune response mediated primarily by cytotoxic T-cells, or a humoral immune response mediated primarily by helper T-cells, which in turn activates B-cells leading to antibody production.

As used herein, the term "naked DNA" refers to a plasmid comprising a nucleotide sequences encoding an agent of the present invention together with a short promoter region to control its production. It is called "naked" DNA because the plasmids are not carried in any delivery vehicle. When such a DNA plasmid enters a host cell, such as a eukaryotic cell, the proteins it encodes are transcribed and translated within the cell.

The term "plasmid" as used herein refers to any nucleic acid encoding an expressible gene and includes linear or circular nucleic acids and double or single stranded nucleic acids. The nucleic acid can be DNA or RNA and may comprise modified nucleotides or ribonucleotides, and may be chemically modified by such means as methylation or the inclusion of protecting groups or cap- or tail structures.

The term "vaccine" as used herein refers to a composition which prevents or reduces the risk of infection or which ameliorates the symptoms of infection. The protective effects of a vaccine composition against a pathogen are normally achieved by inducing in the subject an immune response, either a cell-mediated or a humoral immune response or a combination of both. Generally speaking, abolished or reduced incidences of BVDV infection, amelioration of the symptoms, or accelerated elimination of the viruses from the infected subjects are indicative of the protective effects of a vaccine composition. The vaccine compositions of the present invention provide protective effects against infections caused by BVD viruses.

The term "vector;" as used herein, means a tool that allows or facilitates the transfer of a nucleic acid from one environment to another. In accordance with the present invention, and byway of example, some vectors used in recombinant DNA techniques allow nucleic acids, such as a segment of DNA (such as a heterologous DNA segment, for example, a heterologous cDNA segment), to be transferred into a host or a target cell for the purpose of replicating the nucleic acids and/or expressing proteins encoded by the nucleic acids. Examples of vectors used in recombinant DNA techniques include but are not limited to plasmids, chromosomes; artificial chromosomes and viruses.

### DETAILED DESCRIPTION OF THE INVENTION

The following detailed description is provided to aid those skilled in the art in practicing the present invention.

Standard procedures can be used to propagate and purify a plasmid useful in the present invention. The preferred prokaryotic host cell for plasmid propagation is *E. coli* GM2163 cell line, but some other cell types can also be used. RNA transcribed from the plasmid can be introduced by transfection into eukaryotic host cells capable of supporting virus production, such as MDBK cells. The virus can be produced in such host cells and isolated therefrom in highly purified form using known separation techniques such as sucrose gradient centrifugation.

The present disclosure provides immunogenic compositions in which one or more of the mutant BVD viruses described above have been included.

Another aspect is directed to isolated genomic nucleic molecules of the mutant BVD viruses as described above. Nucleic acid molecules as used herein encompass both RNA and DNA.

In this aspect, the isolated genomic nucleic molecule of a BVD virus contains a genomic sequence of a type I virus wherein at least a portion of the NS3 domain is mutated in the helicase domain.

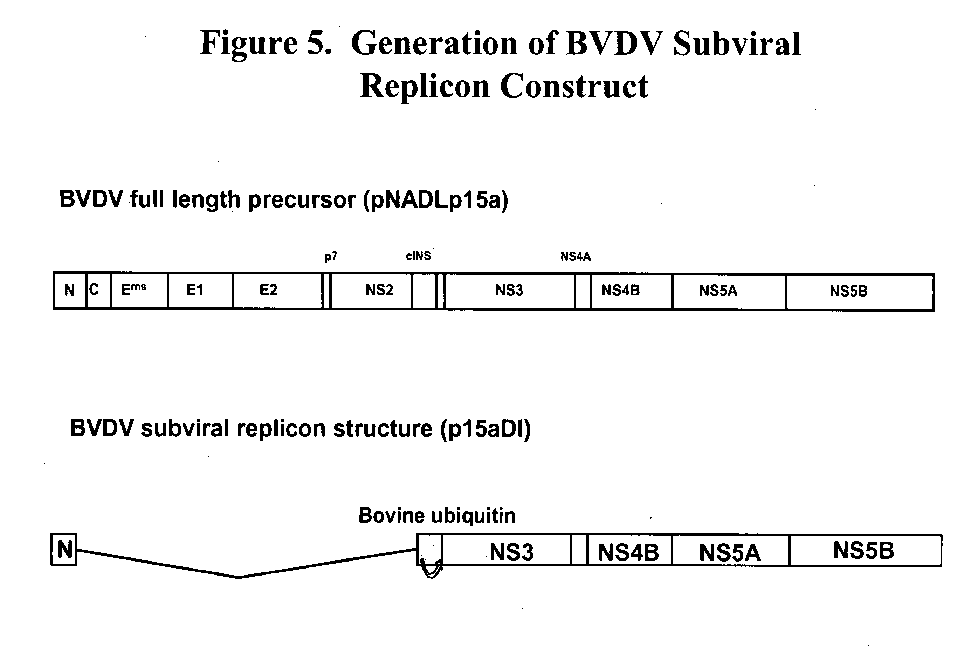

The present disclosure provides vectors in which the genomic nucleic acid sequence of a BVD virus as described herein above has been incorporated. Such vectors can be introduced into appropriate host cells, either for the production of large amounts of the genomic nucleic acid molecules or for the production of progeny mutant BVD viruses. The vectors may contain other sequence elements to facilitate vector propagation, isolation and subcloning; for example, selectable marker genes and origins of replication that allow for propagation and selection in bacteria and host cells. A particularly preferred vector of the present invention is p15aDI (see Fig. 5).

Still another aspect is directed to host cells into which the genomic nucleic acid molecule of a mutated BVD virus of the present invention has been introduced. "Host cells" as used herein include any prokaryotic cells transformed with the genomic nucleic acid molecule, preferably provided by an appropriate vector, of a mutated BVD virus. "Host cells" as used herein also include any eukaryotic cells infected with a mutated BVD virus or otherwise carrying the genomic nucleic acid molecule of a mutated BDV virus. A preferred prokaryotic host cell for plasmid propagation is *E*. *coli* GM2163 cell line, but other cell types can also be used. Preferred eukaryotic host cells include mammalian cells such as MDBK cells (ATCC CCL 22). However, other cultured cells can be used as well. The disclosure further includes progeny virus produced in such host cells.

In another aspect, the viruses may be attenuated by chemical inactivation or by serial passages in cell culture prior to use in an immunogenic composition. The methods of attenuation are well known to those skilled in the art.

The immunogenic compositions disclosed can also include additional active ingredient such as other immunogenic compositions against BVDV, for example, those described in copending U.S. patent application Ser. No. 08/107,908, U.S. Pat. No. 6,060,457, U.S. Pat. No. 6,015,795, U.S. Pat. No. 6,001,613, and U.S. Pat. No. 5,593,873.

In addition, the immunogenic compositions disclosed can include one or more veterinarily-acceptable carriers. As used herein, "a veterinarily-acceptable carrier" includes any and all solvents, dispersion media, coatings, adjuvants, stabilizing agents, diluents, preservatives, antibacterial and antifungal agents, isotonic agents, adsorption delaying agents, and the like. Diluents can include water, saline, dextrose, ethanol, glycerol, and the like. Isotonic agents can include sodium chloride, dextrose, mannitol, sorbitol, and lactose, among others. Stabilizers include albumin, among others. Adjuvants include, but are not limited to, the RIBI adjuvant system (Ribi Inc.), alum, aluminum hydroxide gel, oil-in water emulsions, water-in-oil emulsions such as, e.g., Freund's complete and incomplete adjuvants, Block co polymer (CytRx, Atlanta Ga.), SAF-M (Chiron, Emeryville Calif.), AMPHIGEN® adjuvant, saponin, Quil A, QS-21 (Cambridge Biotech Inc., Cambridge Mass.), or other saponin fractions, monophosphoryl lipid A, Avridine lipid-amine adjuvant, heat-labile enterotoxin from *E*. coli (recombinant or otherwise), cholera toxin, or muramyl dipeptide, among many others. The immunogenic compositions can further include one or more other immunomodulatory agents such as, e.g., interleukins, interferons, or other cytokines.

The immunogenic compositions disclosed can be made in various forms depending upon the route of administration. For example, the immunogenic compositions can be made in the form of sterile aqueous solutions or dispersions suitable for injectable use, or made in lyophilized forms using freeze-drying techniques. Lyophilized immunogenic compositions are typically maintained at about 4° C., and can be reconstituted in a stabilizing solution, e.g., saline or and HEPES, with or without adjuvant.

The immunogenic compositions disclosed can be administered to animal subjects to induce an immune response against BVD viruses. Accordingly, another aspect provides methods of stimulating an immune response against BVD viruses, by administering to an animal subject an effective amount of an immunogenic composition described above.

In accordance with the methods disclosed, a preferred immunogenic composition for administration to an animal subject includes a mutated BVD virus. An immunogenic composition containing a mutated virus, preferably attenuated by chemical inactivation or serial passage in culture, is administered to a cattle preferably via parenteral routes, although other routes of administration can be used as well, such as e.g., by oral, intranasal, intramuscular, intra-lymph node, intradermal, intraperitoneal, subcutaneous, rectal or vaginal administration, or by a combination of routes.

Immunization protocols can be optimized using procedures well known in the art. A single dose can be administered to animals, or, alternatively, two or more inoculations can take place with intervals of two to ten weeks. The extent and nature of the immune responses induced in the cattle can be assessed by using a variety of techniques. For example, sera can be collected from the inoculated animals and tested for the presence of antibodies specific for BVD viruses, e.g., in a conventional virus neutralization assay. Detection of responding CTLs in lymphoid tissues can be achieved by assays such as T cell proliferation, as indicative of the induction of a cellular immune response. The relevant techniques are well described in the art, e.g., Coligan et al. Current Protocols in Immunology, John Wiley &Sons Inc. (1994).

Another aspect is directed to vaccine compositions.

In one aspect, the vaccine compositions disclosed include an effective amount of one or more of the above-described mutated BVD viruses. Purified mutated viruses can be used directly in a vaccine composition, or mutated viruses can be further attenuated by way of chemical inactivation or serial passages in vitro. Typically, a vaccine contains between about 1×10⁶ and about 1×10⁸ virus particles, with a veterinarily acceptable carrier, in a volume of between 0.5 and 5 ml. The precise amount of a virus in a vaccine composition effective to provide a protective effect can be determined by a skilled veterinary physician. Veterinarily acceptable, carriers suitable for use in vaccine compositions can be any of those described hereinabove.

In another aspect, the vaccine compositions as disclosed include the nucleic acid molecule of a mutated virus as claimed. Either DNA or RNA molecules encoding all or a part of the BVD virus genome can be used in vaccines. The DNA or RNA molecule can be present in a "naked" form or it can be administered together with an agent facilitating cellular uptake (e.g., liposomes or cationic lipids). The typical route of administration will be intramuscular injection of between about 0.1 and about 5 ml of vaccine. Total polynucleotide in the vaccine should generally be between about 0.1 µ/ml and about 5.0 mg/ml. Polynucleotides can be present as part of a suspension, solution or emulsion, but aqueous carriers are generally preferred. Vaccines and vaccination procedures that utilize nucleic acids (DNA or mRNA) have been well described in the art, for example, U. S. Pat. No. 5,703,055, U.S. Pat. No. 5,580,859, U.S. Pat. No. 5,589,466.

The vaccine compositions disclosed can also include additional active ingredient such as other vaccine compositions against BVDV, for example, those described in U.S. Pat. No. 6,060,457, U.S. Pat. No. 6,015,795, U.S. Pat. No. 6,001,613, and U.S. Pat. No. 5,593,873.

Vaccination can be accomplished by a single inoculation or through multiple inoculations. If desired, sera can be collected from the inoculated animals and tested for the presence of antibodies to BVD virus.

In another aspect, the above vaccine compositions as disclosed are used in treating BVDV infections. Accordingly, the present disclosure provides methods of treating infections in animal subjects caused by BDV viruses by administering to an animal a therapeutically effective amount of a mutated BVD virus of the present invention.

Those skilled in the art can readily determine whether a genetically engineered virus needs to be attenuated before administration. A mutated virus as disclosed can be administered directly to an animal subject without additional attenuation. The amount of a virus that is therapeutically effective may vary depending on the particular virus used, the condition of the cattle and/or the degree of infection, and can be determined by a veterinarian.

In practicing the present methods, a vaccine composition as disclosed is administered to a cattle preferably via parenteral routes, although other routes of administration can be used as well, such as e.g., by oral, intranasal, intramuscular, intra-lymph node, intradermal, intraperitoneal, subcutaneous, rectal or vaginal administration, or by a combination of routes. Boosting regiments may be required and the dosage regimen can be adjusted to provide optimal immunization.

A further aspect provides methods of determining the attenuated virus of a prior vaccination as the origin of the BVD virus present in an animal subject.

The mutant BVD viruses of the present invention are distinguished from wild type BVD strains in both the genomic composition and the proteins expressed. Such distinction allows discrimination between vaccinated and infected animals, and permits the identification of the BVDV in the event of alleged vaccine-associated outbreaks. For example, a determination can be made as to whether an animal tested positive for BVDV in certain laboratory tests carries a virulent or pathogenic BVD virus, or simply carriers a mutant BVD virus of the present invention previously inoculated through vaccination.

A variety of assays can be employed for making the determination. For example, the viruses can be isolated from the animal subject tested positive for BVDV, and nucleic acid-based assays can be used to determine the presence of a mutant BVD viral genome as indicative of a BVD virus used in a prior vaccination. The nucleic acid-based assays include Southern or Northern blot analysis, PCR, and sequencing. Alternatively, protein-based assays can be employed. In protein-based assays, cells or tissues suspected of an infection can be isolated from the animal tested positive for BVDV. Cellular extracts can be made from such cells or tissues and can be subjected to, e.g., Western Blot, using appropriate antibodies against viral proteins that may distinctively identify the presence of the mutant virus previously inoculated, as opposed to the presence of wild-type BVDV.

### FORMS AND ADMINISTRATION

### PARENTERAL ADMINISTRATION

The compounds of the disclosure may also be administered directly into the blood stream, into muscle, or into an internal organ. Suitable means for parenteral administration include intravenous, intraarterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques.

Parenteral formulations are typically aqueous solutions which may contain excipients such as salts, carbohydrates and buffering agents (preferably to a pH of from 3 to 9), but, for some applications; they may be more suitably formulated as a sterile non-aqueous solution or as a dried form to be used in conjunction with a suitable vehicle such as sterile, pyrogen-free water.

The preparation of parenteral formulations under sterile conditions, for example, by lyophitisation, may readily be accomplished using standard pharmaceutical techniques well known to those skilled in the art.

The solubility of compounds of formula I used in the preparation of parenteral solutions may be increased by the use of appropriate formulation techniques, such as the incorporation of solubility-enhancing agents.

Formulations for parenteral administration may be formulated to be immediate and/or modified release. Modified release formulations include delayed-, sustained-, pulsed-, controlled-, targeted and programmed release. Thus compounds of the disclosure may be formulated as a solid, semi-solid, or thixotropic liquid for administration as an implanted depot providing modified release of the active compound. Examples of such formulations include drug-coated stents and poly(dl-lactic-coglycolic)acid (PGLA) microspheres.

### TOPICAL ADMINISTRATION

The compounds of the disclosure may also be administered topically to the skin or mucosa, that is, dermally or transdermally. Typical formulations for this purpose include gels, hydrogels, lotions, solutions, creams, ointments, dusting powders, dressings, foams, films, skin patches, wafers, implants, sponges, fibres, bandages and microemulsions. Liposomes may also be used. Typical carriers include alcohol, water, mineral oil, liquid petrolatum, white petrolatum, glycerin, polyethylene glycol and propylene glycol. Penetration enhancers may be incorporated - see, for example, Transdermal Penetration Enhancers: Applications, Limitations, and Potential J. Pharm Sci, 88 (10), 955-958, by Finnin and Morgan (October 1999).

Other means of topical administration include delivery by electroporation, iontophoresis, phonophoresis, sonophoresis and microneedle or needle-free (e.g. Powderject™, Bioject™*, etc.)* injection.

Formulations for topical administration may be formulated to be immediate and/or modified release. Modified release formulations include delayed-, sustained-, pulsed-, controlled-, targeted and programmed release.

### INHALED/INTRANASAL/ADMINISTRATION

The compounds of the disclosure can also be administered intranasally or by inhalation, typically in the form of a dry powder (either alone, as a mixture, for example, in a dry blend with lactose, or as a mixed component particle, for example, mixed with phospholipids, such as phosphatidylcholine) from a dry powder inhaler or as an aerosol spray from a pressurised container, pump, spray, atomiser (preferably an atomiser using electrohydrodynamics to produce a fine mist), or nebuliser, with or without the use of a suitable propellant, such as 1,1,1,2-tetrafluoroethane or 1,1,1,2,3,3,3-heptafluoropropane. For intranasal use, the powder may comprise a bioadhesive agent, for example, chitosan or cyclodextrin.

The pressurised container, pump, spray, atomizer, or nebuliser contains a solution or suspension of the compound(s) of the disclosure comprising, for example, ethanol, aqueous ethanol, or a suitable alternative agent for dispersing, solubilising, or extending release of the active, a propellant(s) as solvent and an optional surfactant, such as sorbitan trioleate, oleic acid, or an oligolactic acid.

Prior to use in a dry powder or suspension formulation, the drug product is micronised to a size suitable for delivery by inhalation (typically less than 5 microns). This may be achieved by any appropriate comminuting method, such as spiral jet milling, fluid bed jet milling, supercritical fluid processing to form nanoparticles, high pressure homogenisation, or spray drying.

Capsules (made, for example, from gelatin or hydroxypropylmethylcellulose), blisters and cartridges for use in an inhaler or insufflator may be formulated to contain a powder mix of the compound of the disclosure, a suitable powder base such as lactose or starch and a performance modifier such as *I*-leucine, mannitol, or magnesium stearate. The lactose may be anhydrous or in the form of the monohydrate, preferably the latter. Other suitable excipients include dextran, glucose, maltose, sorbitol, xylitol, fructose, sucrose and trehalose.

A suitable solution formulation for use in an atomiser using electrohydrodynamics to produce a fine mist may contain from 1 µg to 20 mg of the compound of the invention per actuation and the actuation volume may vary from 1 µl to 100 µl. A typical formulation may comprise a compound of formula I, propylene glycol, sterile water, ethanol and sodium chloride. Alternative solvents which may be used instead of propylene glycol include glycerol and polyethylene glycol.

Suitable flavours, such as menthol and levomenthol, or sweeteners, such as saccharin or saccharin sodium, may be added to those formulations disclosed intended for inhaled/intranasal administration.

Formulations for inhaled/intranasal administration may be formulated to be immediate and/or modified release using, for example, PGLA. Modified release formulations include delayed-, sustained-, pulsed-, controlled-, targeted and programmed release.

In the case of dry powder inhalers and aerosols, the dosage unit is determined by means of a valve which delivers a metered amount. Units in accordance with the invention are typically arranged to administer a metered dose or "puff" containing from 10 ng to 100 µg of the compound of formula I. The overall daily dose will typically be in the range 1 µg to 100 mg which may be administered in a single dose or, more usually, as divided doses throughout the day.

### RECTAL/INTRAVAGINAL ADMINISTRATION

The compounds of the disclosure may be administered rectally or vaginally, for example, in the form of a suppository, pessary, or enema. Cocoa butter is a traditional suppository base, but various alternatives may be used as appropriate.

Formulations for rectal/vaginal administration may be formulated to be immediate and/or modified release. Modified release formulations include delayed-, sustained-, pulsed-, controlled-, targeted and programmed release.

### OCULAR/AURAL ADMINISTRATION

The compounds of the disclosure may also be administered directly to the eye or ear, typically in the form of drops of a micronised suspension or solution in isotonic, pH-adjusted, sterile saline. Other formulations suitable for ocular and aural administration include ointments, biodegradable *(e.g.* absorbable gel sponges, collagen) and non-biodegradable (*e.g*. silicone) implants, wafers, lenses and particulate or vesicular systems, such as niosomes or liposomes. A polymer such as crossed-linked polyacrylic acid, polyvinylalcohol, hyaluronic acid, a cellulosic polymer, for example, hydroxypropylmethylcellulose, hydroxyethylcellulose, or methyl cellulose, or a heteropolysaccharide polymer, for example, gelan gum, may be incorporated together with a preservative, such as benzalkonium chloride. Such formulations may also be delivered by iontophoresis.

Formulations for ocular/aural administration may be formulated to be immediate and/or modified release. Modified release formulations include delayed-, sustained-, pulsed-, controlled-, targeted, or programmed release.

### KIT-OF-PARTS

Inasmuch as it may desirable to administer a combination of active compounds, for example, for the purpose of treating a particular disease or condition, it is an aspect of the disclosure that two or more pharmaceutical compositions, at least one of which contains a vaccine in accordance with the disclosure, may conveniently be combined in the form of a kit suitable for co-administration of the compositions.

Thus the kit of the disclosure comprises two or more separate pharmaceutical compositions, at least one of which contains a vaccine in accordance with the disclosure, and means for separately retaining said compositions, such as a container, divided bottle, or divided foil packet. An example of such a kit is a syringe and needle, and the like.

The kit of the disclosure is particularly suitable for administering different dosage forms, for example, oral and parenteral, for administering the separate compositions at different dosage intervals, or for titrating the separate compositions against one another. To assist a veterinarian, the kit typically comprises directions for administration.

The present invention is further illustrated by, but by no means limited to, the following examples.

### Examples

### Example 1. Epitope Mapping of NS3 Domains

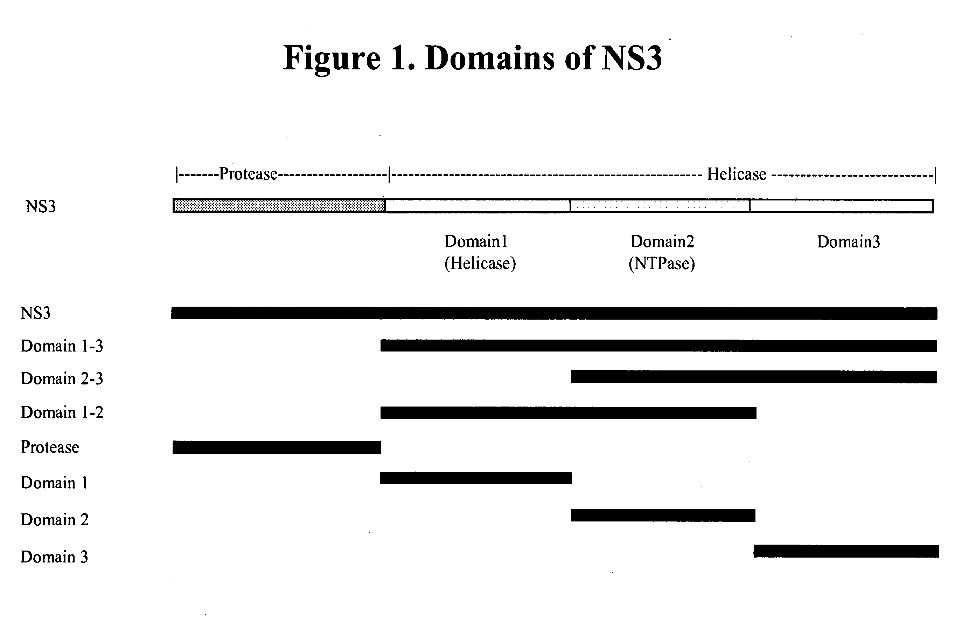

An epitope mapping method was applied to identify the specific epitopes recognized in the NS3 protein by a panel of mAbs. The method entails PCR amplification of each test fragment, followed by translation of the truncated protein *in vitro,* and finally testing of its reactivity with various mAbs. To preliminarily identify antigenic regions on NS3, a set of seven DNA fragments representing the region were amplified (Figure 1). Each fragment contained at its 5' end a T7 promoter followed by an initiation codon, and a stop codon at the 3' end. These DNA fragments were used as template for the generation of S³⁵-labeled protein fragments by *in vitro* transcription/translation using the TnT^{®} Rabbit Reticulocyte Lysate System (Promega; Madison, WI) and radio-labeled methionine and cysteine. The resulting translated protein fragments included full-length NS3 protein, helicase, and protease, as well as individual subdomains of the helicase. (The boundaries of the protease, helicase and helicase subdomains were identified based on sequence alignment of the BVDV and HCV NS3 proteins.) A panel of 12 mAbs recognizing BVDV NS3, including several used by diagnostic laboratories for the detection of BVDV infection in cattle, were used to immunoprecipitate the translated proteins. These monoclonal antibodies are known in the art, and described as being "previously prepared" in Deregt et al., Mapping of two antigenic domains on the NS3 protein of the pestivirus bovine viral diarrhea virus, Veterinary Microbiology (2005), 108(1-2), 13-22. The immunoprecipitates were then analyzed by SDS-PAGE and fluorography.

The results of the immunoprecipitation are summarized in Table 1. All 12 mAbs and the polyclonal serum (POLY) recognized full length NS3, and one or more helicase subdomains, while none recognized the protease fragment. Three mAbs (1.11.3, 21.5.8, and 24.8) immunoprecipitated both the full-length helicase and domain 1-domain 2 (d1-d2) fragment but not the d2-d3 fragment, suggesting that these three antibodies recognize domain 1 of the helicase protein. Both mAbs 21.5.8 and 24.8 bound to the d1 fragment, but mAb 1.11.3 did not, suggesting that the 1.11.3 antibody was more sensitive to epitope conformation than either of the 21.5.8 and 24.8 mAbs. MAb 2.32.5 recognized both the full length helicase and to some extent the d1-d2 fragment, but not the d2-d3 fragment, suggesting that it may also recognize domain 1. Weak binding of the d1-d2 fragment may indicate that the epitope recognized by 2.32.5 differs between the d1-d2 fragment and full-length helicase. MAbs 4.11.4 and 16.1.5 bound both the full-length NS3 and helicase, but only weakly to the d1-d2 and d2-d3 fragments, suggesting they may be specific for an epitope within the second domain of the helicase. Four mAbs, 5.2.1, 9.10.4, 12.7.3 and 15.14.6 recognize both full-length NS3 and the helicase. They also weakly bound to the d2-d3 fragment, but not the d1-d2 fragment, suggesting that they recognize epitopes located in domain 3. That none of them bound to the single d3 fragment suggests that proper folding of d3 may not occur in the absence of the other subdomains. MAb19.7.6 bound to NS3 and the full-length helicase, but not to any of the other fragments. Recognition by this antibody may require the presence of the intact helicase protein. MAb 20.10.6 bound to NS3, the full-length helicase, and both the d1-d2 and d2-d3 fragments very well. It also recognized the single d2 fragment, suggesting that the epitope in domain 2 recognized by this antibody is not affected by the absence of domains 1 and 3. That none of the 12 mAbs bound to full-length protease was not surprising, as even the polyserum (POLY) from a BVDV-infected cow did not recognize the protease in our experiments, strongly suggesting that the protease is not very antigenic. This is consistent with both the molecular orientation of the protease, helicase, and NS4A (protease cofactor) proteins in HCV, in that the orientation of the protease between the helicase and NS4A proteins leaves very little of its surface structure accessible to antibody binding. Based on these results domain 1 is an exemplary target for introduction of a mutation(s) resulting in a marked virus.

**Table 1. Immunoprecipitation of NS3 Subdomains**

| | 1.11.3 | 2.32.5 | 4.11.4 | 5.2.1 | 9.10.4 | 12.7.3 | 15.14.6 | 16.1.5 | 19.7.6 | 21.5.8 | 24.8 | 20.10.6 | POLY |
|---|---|---|---|---|---|---|---|---|---|---|---|---|---|
| NS3 | + | ++ | ++ | ++ | ++ | + | ++ | + | + | ++ | ++ | ++ | ++ |
| Domain 1-3 | ++ | ++ | ++ | ++ | ++ | ++ | ++ | + | + | ++ | ++ | ++ | ++ |
| Domain 1-2 | ++ | +/- | +/- | - | - | - | - | +/- | - | ++ | ++ | ++ | ++ |
| Domain 2-3 | - | - | +/- | +/- | +/- | +/- | +/- | +/- | - | - | - | ++ | ++ |
| Protease | - | - | - | - | - | - | - | - | - | - | - | - | - |
| Domain 1 | | - | - | - | - | - | - | - | - | ++ | ++ | - | +/- |
| Domain 2 | - | - | - | - | - | - | - | - | - | - | - | + | + |
| Domain 3 | - | - | - | - | - | - | - | - | - | - | - | - | +/- |
| Epitope | d1 | d1 | d2-d3 | d3 | d3 | d3 | d3 | d2-d3 | d1 | d1 | d1 | d2 | NS3 |

### Example 2. Sequence Alignment of BVDV and HCV Helicases

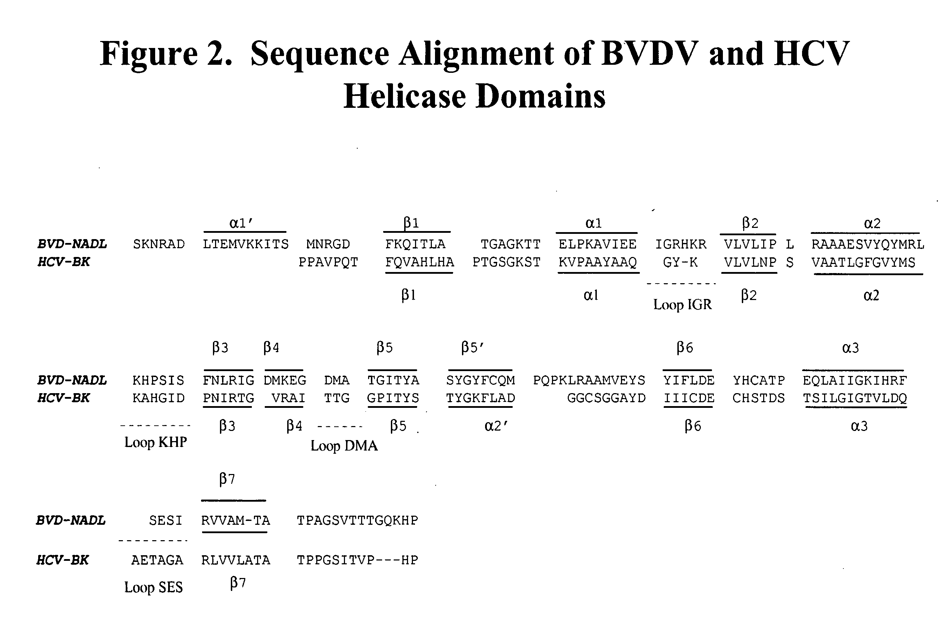

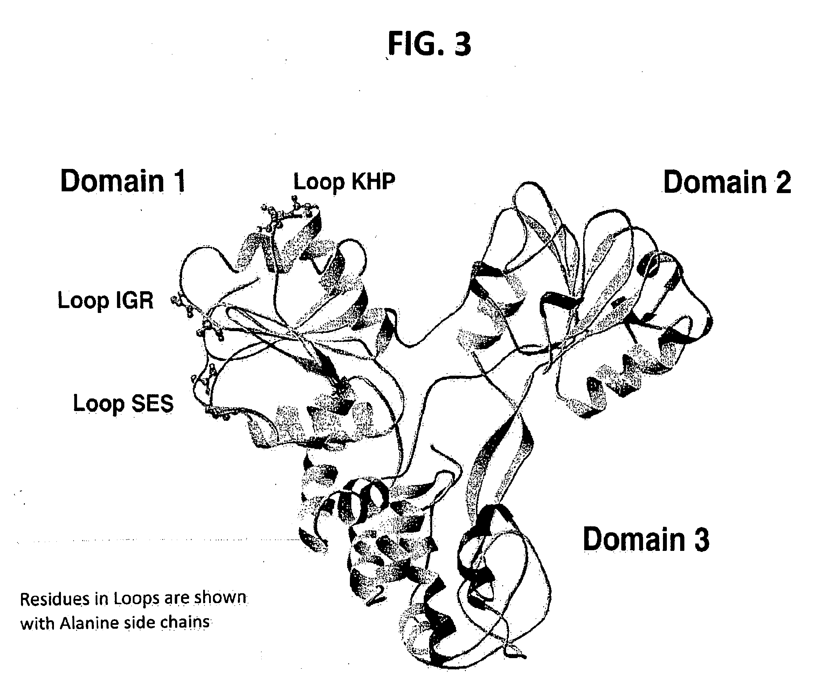

In order to generate a marked virus based on a mutation within domain 1 of the BVDV helicase, further refinement of the epitopes within this domain is desirable. It is desirable to delete an immunodominant epitope without significantly altering the function of the helicase. In order to facilitate the identification of candidate epitopes to mutate, a molecular model of the BVDV helicase would be extremely useful. Since the crystal structure of the HCV helicase is known, it can be used as a template for modeling. To begin the process of generating a molecular model of domain 1, the amino acid sequences of domain 1 of the BVDV and HCV helicases were aligned. The primary sequence identity between them is about 34%. To elucidate the secondary structure of the BVDV helicase domain 1, 47 NS3 sequences derived from various BVDV isolates and other pestivirus were aligned using the Pileup program from the Genetics Computer Group software package (University of Wisconsin; Madison, WI), and the NADL BVDV strain as prototypical sequence. From the aligned sequences, a multiple sequence file (MSF) was generated, and submitted to the JPred server (Cuff, et al., Bioinformatics, 14:892-893 (1998)) for secondary structure prediction using the PHD prediction method (Rost and Sander, J. Mol. Biol. 235:13-26 (1993). A Silicon Graphics Indigo2 Impact 10000 workstation (Silicon Graphics; Mountain View, CA) was used for all molecular modeling studies. The Molecular Operating Environment (MOE) version 2001.01 (Chemical Computing Group, Inc.; Montreal, Quebec) and SYBYL 6.7 software (Tripos Associates Inc.; St. Louis, MO) were used for molecular modeling and visualizations. The amino acid sequences of domain1 and 2 from the HCV (SEQ ID NO. 21) and BVDV (SEQ ID NO. 20) NS3 proteins were aligned (Figure 2) based on the primary sequence homology and secondary structure predictions. A preliminary molecular model of the BVDV NS3 domain 1 and 2 was then generated, using the corresponding region of the HCV protein as template. As shown in Figure 3, the presence of several loops and turns between the alpha helices and beta strands, including α1-β2 (Loop IGR), α2-β3 (KHP), β4-β5 (DMA) and α3-β7 (SES), leads to the formation of an exposed surface away from both the helicase catalytic center and the helicase-protease interactive surface. This area has the potential to be a highly antigenic region. Three of the loops identified, Loop KHP, Loop IGP, and Loop SES, were chosen as targets for a mutagenesis study.

### Example 3. Location of mAb Binding Sites by Scanning Mutagenesis

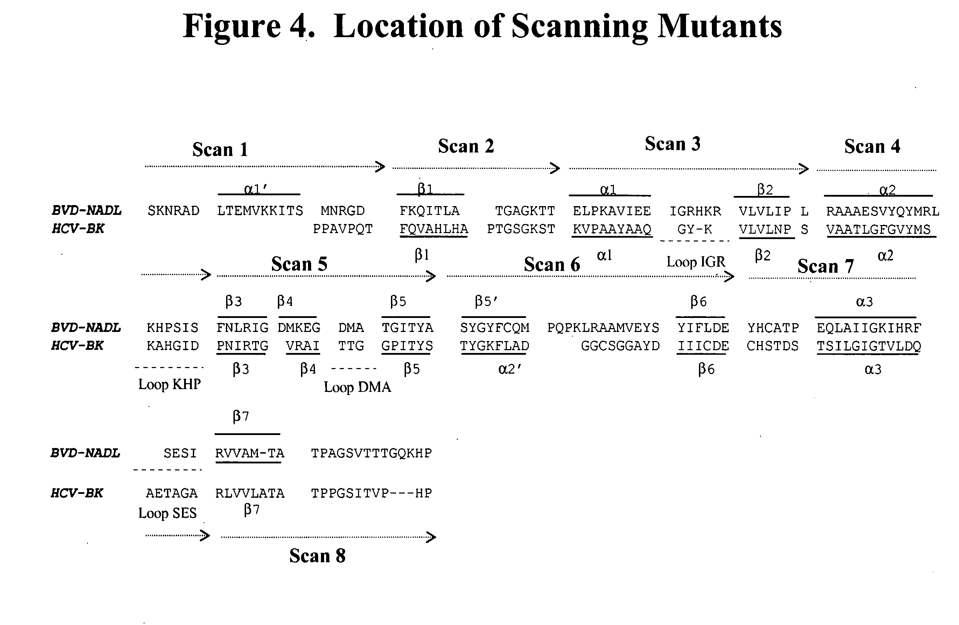

To further define epitopes in domain 1 bound by various mAbs, a scanning mutagenesis method was employed. Briefly, short segments of the BVDV helicase domain 1 sequence (SEQ ID NO. 20) were replaced with the corresponding HCV sequence (SEQ ID NO 21) using PCR amplification, followed by restriction enzyme digestion and ligation of the resulting fragments, generating the "scanning mutants" indicated in Figure *4**. In vitro* transcription and translation, as well as immunoprecipitation, was carried out as described in Example 1. A summary of reactivity of the various mAbs with the mutants is shown in Table 2.

**Table 2. Reactivity of Scanning Mutants with mAbs**

| **mAbs** | **Scan** | **Scan** | **Scan** | **Scan** | **Scan** | **Scan** | **Scan** | **Helicase** |
|---|---|---|---|---|---|---|---|---|
| 1.11.3 | ++ | - | ++ | - | - | - | + | +++++ |
| 21.5.8 | ++ | - | +/- | - | +/- | + | ++ | +++++ |
| 24.8 | ++ | + | - | - | - | +/- | ++ | +++++ |
| 20.10.6 | ++++ | +++ | ++++ + | ++ | ++ | ++ | ++ | +++++ |
| Poly serum | ++++ + | ++++ | ++++ + | ++ | ++ | ++ | +++ | +++++ |
| CA72 negativ | - | - | - | - | - | - | - | - |

### Example 4. Detailed Resolution of mAb Binding Sites by Alanine Replacement Mutagenesis

To further define the epitopes in domain 1 bound by various mAbs, and to identify the critical residues in these regions for antibody binding, a total of sixteen single amino acid (alanine) replacement mutants in three regions, I1841-R1846, K1867-S1872 and S1938-I1941 were generated and tested for antibody binding. Amino acid residue coordinates are according to SEQ ID NO. 1. Thus, "I1841A" represents a replacement of Isoleucine with Alanine at coordinate 1841 as numbered in SEQ ID NO. 1. Of course, in other BVDV isolates, different specific amino acids may be present at the particular coordinates of the exemplary sequence. Therefore, a mutation at the same locus of the helicase domain of a variant BVD virus, or plasmid constructed to express a variant BVD virus, will result in an equivalent loss of recognition by antibodies raised against the variant, unmodified virus peptide. The replacement mutants were constructed using a PCR overlap extension technique known in the art (see for example, Ho et al., Gene, 77(1):51-9 (1989)). Briefly, PCR was used to generate the alanine replacement fragments, each encoding domain 1 and 2 of the helicase. Each fragment encoded a T7 promoter sequence and translation initiation codon at its 5' end, and a stop codon at the 3' end. Initially, two separate reactions were carried out to generate overlapping fragments encoding the 5' and 3' halves of the replacement region. Within the region of overlap, a single alanine mutation was introduced into the sequence of both fragments by virtue of mutagenic oligonucleotide primers used in the PCR. The products of each PCR were separated by electrophoresis in an agarose gel, and a single band of the correct size was purified from each reaction. The purified DNA fragments were mixed and used as templates for a second PCR to generate a single replacement fragment. This entire procedure was repeated to generate each of the desired replacement fragments. The sequence of each fragment was verified by DNA sequencing. S³⁵-labeled protein fragments were generated using these fragments as template via *in vitro* transcription/translation as described above. Immunoprecipitation using mAbs, followed by SDS-PAGE analysis, was employed to determine if the mutated epitopes were still recognized by the antibodies.

E1939A and R1942A, completely disrupted binding by mAb 1.11.3, suggesting that these two residues are crucial for antibody binding. That these two amino acids are on the same α3-β7 (SES) loop (Figure 3) suggests that the epitope recognized by this antibody is formed by this loop. Two other mutants, I1841A and K1867A, which are located on two separated regions of the helicase molecule (α1-β2 (IGR) and α2-β3 (KHP) loops), displayed significantly reduced binding by mAb 21.5.8, but not the other antibodies. One conclusion that could be drawn from these results would be that the epitope recognized by this mAb might encompass two different loops which are located in close proximity in the native molecule. This is consistent with the molecular model shown in Figure 3. The mutant R1843A destroyed binding by mAb 24.8, but had no effect on binding of the other antibodies. Again, this would suggest that this residue is part of a key epitope located on the α1-β2 (IGR) loop. The partial effect of the R1942A mutant on binding of mAb 24.8 suggests that the α3-β7 (SES) loop, together with the α1-β2 (IGR) loop, constitutes the epitope recognized by this antibody. In conclusion, the epitopes recognized by three mAbs were precisely mapped within domain 1 of the BVDV helicase. Key residues within those epitopes were identified, being located within three separate regions of the primary sequence, but in close proximity in the tertiary conformation. The function of these epitopes were further examined in the context of a BVDV subviral replicon.

**Table 3. Immunoprecipitation of Alanine Replacement Mutants**

| | mAb 1.11.3 | mAb 21.5.8 | mAb 24.8 | Poly serum |
|---|---|---|---|---|
| I1841A | + | + | ++ | ++ |
| R1843A | ++ | + | - | ++ |
| H1844A | ++ | + | ++ | ++ |
| K1845A | + | + | ++ | ++ |
| R1846A | ++ | + | ++ | ++ |
| S1938A | ++ | + | ++ | ++ |
| E1939A | - | + | ++ | ++ |
| S1940A | ++ | + | ++ | ++ |
| I1941A | + | + | ++ | ++ |
| R1942A | - | + | +/- | + |
| K1867A | ++ | + | ++ | ++ |
| H1868A | + | + | ++ | ++ |
| P1869A | ++ | + | + | ++ |
| S1870A | ++ | + | ++ | ++ |
| I1871A | ++ | + | ++ | ++ |
| S1872A | ++ | + | ++ | ++ |

### Example 5. Construction of Helicase Domain 1 Mutations in the Context of a Subviral BVDV Replicon

### Construction of Subviral Replicon

A desireable quality for production of a successful virus vaccine is the ability to obtain high titer virus yields. Therefore, a marker mutation should not interfere significantly with virus replication. As helicase activity is essential for replication of the BVDV RNA, we wanted to assess all domain 1 point mutants made, for not only loss of antibody recognition, but also preservation of catalytic helicase activity. Amplification and genetic manipulation of a full-length BVDV proviral molecular clone in *Escherichia coli (E. coli*) is difficult because the plasmid is unstable during propagation. Therefore, p15aDI, which contains a truncated subviral replicon expressing NS3 and supporting viral RNA replication, yet lacks the viral structural genes, was created to facilitate screening of the mutants. p15aDI was derived from an infectious proviral parent plasmid (pNADLp15a) containing the full-length BVDV genome. More manipulable because it lacks most of the structural genes and the NS2 coding region, the only sequence located upstream of NS3 consists of a fusion between a portion of the N protein to bovine ubiquitin (Figure 5). NS3 protein expressed from this replicon is detectable by immunohistochemistry only when efficient RNA replication leads to the amplification of transcripts, resulting in an increase in viral protein expression. Thus, detection of NS3 serves as indirect confirmation of efficient RNA replication and catalytic helicase activity.

### Generation of BVDV Helicase Domain 1 Mutants

A set of twelve different helicase domain 1 mutants were generated in the context of the subviral replicon, and analyzed for viral RNA replication and loss of epitope recognition. Eight of these mutants contained only a single amino acid change, and included: within the IGR loop, I>A (amino acid residue 1841), R>A (1843), and K>A (1845); within the KHP loop, K>A (1867), H>A (1868), and P>A (1869); within the SES loop, E>A (1939), and R>A (1942). Two mutants had changes in two amino acids: within the IGR loop, R>A (1843) and K>A (1845), and within the SES loop, E>A (1939), and R>A (1942). Two contained three changes: K>A (1867), H>A (1868), and P>A (1869), all within the IGR loop, and K>A (1845), H>A (1868), and E>A (1939), affecting multiple loops. While alanine was used in the exemplary mutations for convenience, non-conservative amino acid substitutions may be utilized as appropriate mutations. Each mutant was generated using the overlapping PCR strategy described above. A specific set of overlapping primers was designed for each desired mutation (Table 4). For screening purposes, each primer set also contained additional silent nucleotide changes, which would result in the creation of a unique novel restriction enzyme cleavage site near the site of the mutation. The overlapping PCR fragments served as templates in the second round of amplification, carried out using only the two outside primers. To generate fragments containing multiple amino acid changes, the amplification reaction was repeated, using the previous mutant fragment as template. The fully mutated fragment was then cloned into the subviral replicon backbone by means of two unique restriction enzyme sites *(Bsm* B I and *Sma* I) created during the PCR process. The mutant PCR fragment and the subviral replicon backbone were both digested with *Bsm* B I and *Sma* I, treated with alkaline phosphatase (NEB, Inc.), purified by agarose gel electrophoresis, and ligated overnight at 16°C using T4 DNA ligase (New England Biolabs, Inc., Beverly, MA). STBL2 *E. coli* cells (Invitrogen; Carlsbad, CA) were transformed with an aliquot of the ligated reaction, and plated on selective media. Colonies were screened by purification of plasmid DNA, followed by digestion with restriction enzymes. Plasmids of the expected size were further confirmed by sequence analysis.

**Table 4**

| **SEQ ID NO** | **UTILITY OF PRIMER** | **SEQUENCE (5'-3')** |
|---|---|---|
| 2 | Flanks 5' end of p15aDI cloning site for mutant fragments | GAGGCCGTTAACATATCA |
| 3 | Flanks 3' end of p15aDI cloning site for mutant fragments | CCTAAATCACTTTGACCCTGTTGCTGT |
| 4 | 5' primer for introducing I1841A mutation | GAGGCAGGGCGCGACAAGAGAGTATTAGTT |
| 5 | 3' primer for introducing I1841A mutation | CTTGTGGCGCCCTGCCTCCTCTATAACTGCTT |
| 6 | 5' primer for introducing R1843A mutation | GAGATAGGCGCCCACAAGAGAGTATTAGTT |
| 7 | 3' primer for introducing R1843A mutation | CTTGTGGGCGCCTATCTCCTCTATAAC |
| 8 | 5' primer for introducing K1845A mutation | ATAGGGCGCCACGCGAGAGTATTAGTTCTTAT |
| 9 | 3' primer for introducing K1845A mutation | TCTCGCGTGGCGCCCTATCTCCTCTATAAC |
| 10 | 5' primer for introducing K1867A mutation | TTGGCTCACCCATCGATCTCTTTTAACCTAAGGA |
| 11 | 3' primer for introducing K1867A mutation | AGAGATCGATGGGTGAGCCAATCTCATATACTGGTAG |
| 12 | 5' primer for introducing H1868A mutation | AAAGCTCCATCGATCTCTTTTAACCTAAGGA |
| 13 | 3' primer for introducing H1868A mutation | AGAGATCGATGGAGCTTTCAATCTCATATACTGG |
| 14 | 5' primer for introducing P1869A mutation | CACGCGAGCATAAGCTTTAACCTAAGGATAGGGG |
| 15 | 3' primer for introducing P1869A mutation | TTAAAGCTTATGCTCGCGTGTTTCAATCTCATATAC |
| 16 | 5' primer for introducing E1939A mutation | CCATCGATTTTCAGCGAGTATAAGGGTTGTCG |
| 17 | 3' primer for introducing E1939A mutation | CTCGCTGAAAATCGATGGATCTTCCCGATAAT |
| 18 | 5' primer for introducing R1942A mutation | CCATCGATTTTCAGAGAGTATAGCGGTTGTCGCCATGACTGC |
| 19 | 3' primer for introducing R1942A mutation | ACCGCTATACTCTCTGAAAATCGATGGATCTTCCCGATAAT |

### Example 6. Characterization of Mutant Subviral Replicons

### In vitro Transcription and RNA Transfection

RNA transcripts were synthesized *in vitro* using T7 RNA polymerase and MEGAscript™ (Ambion; Austin, TX). DNA templates were linearized with *Ksp* I and treated with T4 DNA polymerase to remove the 3' overhang. The products of the transcription reaction were analyzed by agarose gel electrophoresis prior to transfection. 1-5 µg of RNA was added to 200 µl of Opti-MEM (Invitrogen) containing 6 µg of Lipofectin (Invitrogen), and incubated for 10 to 15 min at room temperature. Simultaneously, monolayers (50 to 60% confluent) of Madin Darby Bovine Kidney (MDBK) cells grown in six-well plates (35mm diameter) were washed twice with RNase-free PBS, and once with Opti-MEM. After the final wash, the transfection mixtures were added to each well, followed by incubation for 10 min at room temperature with gentle rocking. 1 ml of Opti-MEM was then added to each well, and plates were incubated for a further 3 hrs at 37°C. Three ml of Opti-MEM containing 2-3% bovine donor calf serum was then added to each of the wells.

### Analysis of RNA Replication and Antibody Recognition

Following incubation for 24-48 hrs at 37°C, the transfected cells were fixed with 80% acetone, and subjected to an immunohistochemistry assay (IHC), using a Vectastain *Elite* ABC kit (Vector Laboratories; Burlingame, CA) according to the manufacturer's instructions. Monoclonal antibody 20.10.6, which recognizes helicase domain 2, was used to visualize cells positive for NS3, as indicator of efficient RNA replication. Cells transfected with wild-type BVDV RNA, as well as many of the mutant replicons, showed strong staining with mAb 20.10.6, indicating that those individual mutant viral helicases supported efficient vRNA replication. Only mutant K1867A/H1868A/P1869A failed to produce detectable NS3 protein, suggesting that this set of mutations significantly interfered with viral RNA replication.

All cells transfected with wild-type or mutant replicons were also tested with mAbs 1.11.3, 21.5.8, and 24.8. (Table 5). Each loop appeared to be recognized by one of these three antibodies, as mutations in each loop resulted in loss of recognition by one of the three antibodies. In particular, mutation of residues R1843A and K1845A in loop IGR, individually and together, resulted in complete loss of recognition by mAb 24.8. At the same time, recognition by mAbs 20.10.6, 1.11.3 and 21.5.8 was not affected. In loop KHP, mutation K1867A abolished recognition by mAb 21.5.8, without affecting recognition by the other three antibodies. Also, both point mutations in loop SES lead to a loss of recognition by mAb 1.11.3, as did the double mutant. Additionally, the triple mutant (K1845A/H1868A/E1939A) resulted in a loss of recognition by both 1.11.3 and 24.8 mAbs, while antibody recognition by mAbs 20.10.6 and 21.5.8 was not affected.

In summary, several mutations in the three helicase loops that resulted in abolishment of mAb recognition and binding were identified. In addition, it was found that it is feasible to simultaneously disrupt recognition sites for two antibodies, while still maintaining helicase function. Thus, each of these individual mutations, or a combination of them, could serve as a marked BVDV vaccine, containing a mutation(s) within the helicase region.

**Table 5. Immunoreactivity of mAbs with Helicase Mutants**

| | Monoclonal Antibody | | | |
|---|---|---|---|---|
| Mutation | 20.10.6 | 1.11.3 | 21.5.8 | 24.8 |
| WT/DI | +++ | ++/+++ | ++/+++ | +++ |
| | | | | |

| Loop IGR | | | | |
|---|---|---|---|---|
| l1841A | +++ | ++/+ | +/- | +++ |
| R1843A | +++ | ++ | ++ | - |
| K1845A | +++ | ++/+ | ++ | - |
| RK1843/45 | +++ | ++/+ | +++ | - |
| A | | | | |
| Loop KHP | | | | |
| K1867A | +++ | ++ | - | + |
| H1868A | +++ | ++ | ++ | ++/+ |
| P1869A | +++ | ++/+++ | +++ | +++ |
| KHP1867/68/69 | - | | | |
| A | | | | |

| Loop SES | | | | |
|---|---|---|---|---|
| E1939A | +++ | - | ++ | +++ |
| R1942A | +++ | - | ++ | +++ |
| ER1939/42A | +++ | - | +/- | ++/+++ |
| | | | | |

| Multiple Loops | | | | |
|---|---|---|---|---|
| K1845A-H1868A-E1939A | +++ | - | | - |
| K1845A-KHP1868FAS- | | | +/++ | |
| ER1939A | | | | |

### Example 7. Generation and Analysis of Marked Viruses

In order to evaluate the effect(s) of directed mutations within the NS3 protein on viral replication and infectivity, it was necessary to move the mutations into a proviral plasmid containing the full-length BVDV sequence (pNADLp15A). The three mutated sequences chosen for further study were: K1845A-H1868A-E1939A, R1942A, and E1939A. A DNA fragment containing each respective mutated sequence of interest was cloned into pNADLp15A, once again utilizing the unique *Bsm* BI and *Sma* I restriction sites. The ligation mixtures were transformed into *E*. *coli* GM2163 cells (New England Biolabs, Inc.; Beverly, MA), and then plated on selective media. Following overnight incubation, colonies were screened for the presence of plasmid containing the correct sequence. One clone representing each mutation was selected (R1942A; E1939A; and K-H-E), and from these clones, viral RNA was prepared as described in Example 6. MDBK cells were transfected with each RNA preparation, and incubated at 37C° for 64 hours. Duplicate transfections of RD cells (ATCC; Rockville, MD) were set up for each mutant. One set of transfected cells was fixed for IHC staining as described in Example 6, and from the second set, cells were scraped from the seeded flasks and stored at -80°C as stocks for future propagations.

In order to further evaluate the virus produced by the three clones, culture fluids harvested from the transfection experiment were passed onto the fresh RD cell monolayers. Following adsorption and overnight incubation, cells were fixed for IHC analysis. The results of that analysis are shown in Table 6. Both the wild-type and mutant viruses were recognized by mAb 20.10.6 (control antibody). The wild-type virus was also recognized by mAbs 1.11.3 and 24.8. Mutant E1939A was bound by mAb 24.8, but not 1.11.3. Mutant K-H-E was recognized only by mAb 20.10.6, and not by 1.11.3 or 24.8. Mutant R1942A demonstrated reactivity with mAb 24.8, but not with 1.11.3.

**Table 6. IHC Analysis of Cells Infected with Marked Viruses**

| | Monoclonal Antibody | | | |
|---|---|---|---|---|
| Mutation | 20.10.6 | 1.11.3 | 21.5.8 | 24.8 |
| | | | | |
| Loop 2 | | | | |
| K1867A | No Virus Growth | | | |
| | | | | |
| Loop 3 | | | | |
| E1939A | +++ | - | ++ | +++ |
| R1942A | +++ | - | ++ | +++ |
| | | | | |
| Multiple Loops | | | | |
| K1845A-H1868A- | +++ | - | +/++ | - |
| E1939A | | | | |

The growth kinetics of each marked virus was also assessed. Stock virus titers for each were pre-determined using a standard virus titration protocol. In a time-course study, fresh monolayers of RD cells were seeded in tissue culture flasks, incubated overnight, and the following day infected with a pre-determined amount of each virus. Following adsorption and washing, an initial set of samples were collected (Hour "0"). Samples were subsequently collected at 14, 19, 24, 39, 43, 47, and 65 hrs post infection. Virus titers were determined using the Spearman-Karber method (Hawkes, R. A. In E. H. Lennette (ed.), Diagnostic Procedures for Viral, Rickettsial and Chlamydial Infections, p. 33-35; 7th ed. American Public Health Association Publications, Washington, D.C.) and expressed as TCID₅₀/ml. Compared to the wild-type (parent) BVD virus, all of the mutants grew at a rate similar to, or in some cases, slightly better than, the wild-type (Table 7).

**Table 7. Comparative Titers of Wild-Type and Mutant BVD Viruses (TCID₅₀/ml)**

| **Hours** | **Wild Type NDAL** | | **K-H-E#9** | | **R1942A#73** | | **E1939A#84** | |
|---|---|---|---|---|---|---|---|---|
| 0 | 0 | 4 | 4 | 2.5 | 0 | 0 | 0 | 0 |
| 14 | 2.5e+3 | 1.6e+3 | 1.0e+1 | 2.5e+1 | 2.5e+2 | 4.0e+2 | 6.3e+2 | 2.5e+3 |
| 19 | 6.3e+3 | 6.3e+3 | 1.0e+3 | 4.0e+3 | 1.6e+3 | 4.0e+3 | 4.0e+3 | 6.3e+3 |
| 24 | 1.6e+4 | 4.0e+4 | N/D | N/D | 1.6e+3 | 6.3e+3 | 2.5e+4 | 2.5e+4 |
| 39 | 4.0e+5 | N/D | N/D | N/D | 6.3e+4 | 1.0e+5 | 1.0e+6 | 4.0e+5 |
| 43 | 2.5e+5 | 6.3e+5 | 6.3e+4 | 6.3e+4 | 1.6e+5 | 1.6e+5 | 1.0e+6 | 2.5e+6 |
| 47 | 1.6e+5 | 5.0e+5 | 1.6e+5 | 2.5e+5 | 2.5e+5 | 4.0e+5 | 1.6e+6 | 4.0e+6 |
| 65 | 1.6e+5 | 2.8e+5 | 4.0e+5 | 2.5e+5 | 2.5e+6 | 2.5e+6 | 6.3e+6 | 1.0e+7 |

Some of the mutations generated resulted in the alteration of specific immunologically distinct epitopes, as determined by a panel of monoclonal antibodies. Similar results were obtained when antibody recognition was analyzed in the context of an infectious viral particle. Clones containing mutations which did not interfere with the generation of infectious virus, yet led to a loss in recognition by mAbs, represent novel strains which serve as effective marked BVDV vaccine strains.

### Example 8. Vaccine Efficacy Testing in a Young Calf Model

BVDV negative healthy calves are obtained, randomly assigned to study groups, and maintained under supervision of an attending veterinarian. The test vaccine is combined with a sterile adjuvant, and administered by either intramuscular (IM) or subcutaneous (SC) injection. Two doses of vaccine are administered, 21 to 28 days apart. The animals are subsequently challenged at 21 to 28 days following the final vaccination with a Type 1 or Type 2 strain of BVDV. Challenge inoculum is given intranasally in a 4 ml divided dose, 2 ml per nostril. Control groups consisting of unvaccinated, unchallenged animals and/or unvaccinated, challenged animals are also maintained throughout the study.

Clinincal parameters are monitored daily, including rectal temperature, depression, anorexia, and diarrhea. Serum neutralization titers are determined by a constant-virus, decreasing-serum assay in bovine cell culture, using serial dilutions of serum combined with a BVDV Type 1 or 2 strain. Post-challenge isolation of BVDV in bovine cell culture is attempted from peripheral blood. A BVDV-positive cell culture is determined by indirect immunofluorescence. To demonstrate protection following challenge, a reduction in incidence of infection has to be demonstrated in vaccinated groups versus the control groups.

### Example 9. Vaccine Efficacy Testing in a Pregnant Cow-Calf Model

BVDV-negative cows and heifers of breeding age are obtained and randomly assigned to a vaccination test group or a placebo (control) group. Cows are inoculated twice by intramuscular (IM) or subcutaneous (SC) injection, with either vaccine or placebo, 21 to 28 days apart. Following the second vaccination, all cows receive an IM prostaglandin injection to synchronize estrus. Cows which display estrus are bred by artificial insemination with certified BVDV-negative semen. At approximately 60 days of gestation, the pregnancy status of cows is determined by rectal palpation. Approximately 6 weeks later, cows with confirmed pregnancies are,randomly selected from each test group. Each of these cows is challenged by intranasal inoculation of BVDV Type 1 or 2. Blood samples are collected on the day of challenge and at multiple postchallenge intervals for purposes of BVDV isolation.

Twenty-eight days after challenge, left flank laparotomies are performed and amniotic fluid is extracted from each cow. Immediately prior to surgery, a blood sample is collected from each cow for serum neutralization assays. Following caesarian delivery, a blood sample is collected from each fetus. Fetuses are then euthanized, and tissues are aseptically collected for purposes of BVDV isolation. In cases where spontaneous abortions occur, blood samples are taken from the dam when abortion is detected and two weeks later. The paired blood samples and aborted fetuses are subjected to serologic testing and virus isolation. Vaccine efficacy is demonstrated by a lack of fetal infection and late-term abortion.

### Example 10. Diagnostic Assays for Marked BVDV Vaccines

Cattle of various ages may be vaccinated with either a live-attenuated or inactivated NS3-mutated (marked) BVDV vaccine according to instructions provided. Serum samples can be collected 2-3 weeks or later following vaccination. To differentiate between cattle, which received the marked BVDV vaccine versus those infected by a field (wild type) strain of BVDV, serum samples may be tested via a differential diagnostic assay. The NS3 protein with epitope-specific amino acid mutations can, when presented to cattle in the context of a marked vaccine, elicit the production of specific antibodies which will bind to the mutated epitopes of NS3 protein, but not to the non-mutated epitopes present on wild type virus. In the context of wild-type virus, the opposite is true- that specific antibodies may recognize the wild-type epitopes on the NS3 protein, but not the mutated form. Methods of assaying for antibody binding specificity and affinity are well known in the art, and include but are not limited to immunoassay formats such as ELISA, competitive immunoassays, radioimmunoassays, Western blots, indirect immunofluorescent assays, and the like.

A competitive ELISA may be an indirect or a direct assay. One example of a direct competitive assay is described herein. Whole or partial wild type viral antigens, including the NS3 protein (naturally or synthetically derived), may be used as an antigen source. Following coating of the ELISA plate with antigen under alkaline conditions, cattle serum samples and dilutions are added together with an optimized dilution of the epitope-specific mAb, and incubated for 30 -90 min. Either horseradish peroxidase or alkaline phosphatase has been conjugated to the mAb to allow for colorimetric detection of binding. Following washing of the plates, an enzyme-specific chromogenic substrate is added, and after a final incubation step, the optical density of each well is measured at a wavelength appropriate for the substrate used. Depending on the level of reactivity of the cattle serum with the NS3 protein coating the plate, binding of the labeled mAb could be inhibited. A lack of binding by the mAb indicates the presence of antibodies in the cattle serum that recognize the wild type-specific epitope, indicative of a natural (wild-type) infection. In contrast, serum from cattle immunized with the marked vaccine possessing an epitope specific mutation(s) will not contain antibodies which will bind to the NS3 protein coating the plate. Therefore, the mAb will bind to the NS3 protein, and result in subsequent color development.

## Claims

1. A bovine viral diarrhea virus comprising at least one helicase domain amino acid mutation, wherein the mutation in the helicase domain of NS3 is selected from the group consisting of:
(a) a helicase domain mutation within the IGR loop at amino acid residue 1845;
(b) a helicase domain mutation within the SES loop at amino acid residue 1939 or 1942; or
(d) a helicase domain mutation within the IGR, KHP and SES loops at amino acid residues 1845, 1868 and 1939, respectively;
and results in a loss of recognition by a monoclonal antibody raised against NS3 from wild-type bovine viral diarrhea virus but wherein viral RNA replication and the generation of infectious virus is retained; and wherein the amino acid residues are numbered according to SEQ ID NO: 1.

## Patentansprüche

1. Bovine-Virusdiarrhoe-Virus, umfassend wenigstens eine Helicase-Domäne Aminosäure-Mutation, wobei die Mutation in der Helicase-Domäne von NS3 ausgewählt ist aus der Gruppe, bestehend aus:
(a) Helicase-Domäne-Mutation in der IGR-Schleife an Aminosäurerest 1845,
(b) Helicase-Domäne-Mutation in der SES-Schleife an Aminosäurerest 1939 oder 1942, oder
(d) Helicase-Domäne-Mutation in den IGR-, KHP- und SES-Schleifen an den Aminosäureresten 1845, 1868 bzw. 1939,
und in einem Verlust der Erkennung durch einen monoklonalen Antikörper, der gegen NS3 aus Wildtyp Bovine-Virusdiarrhoe-Virus gerichtet wurde, resultiert, aber wobei viral RNA-Replikation und die Herstellung des infektiösen Viruses erhalten bleibt, und wobei die Aminosäurereste gemäß SEQ ID NO: 1 nummeriert sind.

## Revendications

1. Virus de la diarrhée virale bovine comprenant au moins une mutation d'acide aminé de domaine hélicase, dans lequel la mutation dans le domaine hélicase de NS3 est choisie parmi le groupe consistant en :
(a) une mutation de domaine hélicase au sein de la boucle IGR au résidu d'acide aminé 1845;
(b) une mutation de domaine hélicase au sein de la boucle SES au résidu d'acide aminé 1939 ou 1942 ; ou
(d) une mutation de domaine hélicase au sein des boucles IGR, KMP et SES respectivement aux résidus d'acides aminés 1845, 1868 et 1939 ;
et résulte en une perte de reconnaissance par un anticorps monoclonal élevé contre NS3 du virus de diarrhée virale bovine de type naturel mais dans lequel la réplication d'ARN viral et la génération de virus infectieux est retenue ; et dans lequel les résidus d'acides aminés sont numérotés selon la SEQ ID NO: 1.
